# EUROPEAN PATENT APPLICATION

(11) **EP 2 213 638 A1**
(43) Date of publication of application: **04.08.2010**
(21) Application number: 09151892.8
(22) Date of filing: 02.02.2009
(51) Int. Cl.: C07B 53/00, C07C 231/16

(54) **Method for the asymmetric hydrogenation of olefins utilising photoisomerisation**

(71) Applicant: DSM IP Assets B.V., 6411 TE Heerlen (NL)
(72) Inventor: The designation of the inventor has not yet been filed
(74) Representative: Duffy, James Edwin Marsh

(57) **Abstract**

The present invention relates to a method for the preparation of a compound of formula (I), 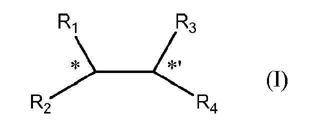 wherein R¹ is different to R²; R³ is different to R⁴; and R¹, R², R³ and R⁴ are each a moiety other than hydrogen, which method comprises,
i) photoirradiating at an intensity above ambient intensity a solution comprising a compound of formula (II), 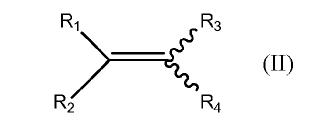 wherein R¹, R², R³ and R⁴ are as defined above, to form a solution of a mixture of compounds of formula (IIa) and (IIb), 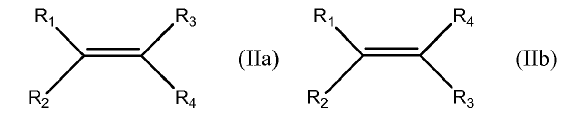 wherein R¹, R², R³and R⁴ are as defined above; and
ii) contacting the mixture of compounds (IIa) and (IIb) with a hydrogen donating agent in the presence of an asymmetric hydrogenation catalyst, after initiation of i) and before termination of i).

## Description

The present invention relates to a method for the asymmetric hydrogenation of a tetrasubstituted olefin, utilising photoisomerisation, to yield a compound having stereogenic centres on two adjacent carbon atoms.

Compounds containing two adjacent stereogenic carbon centers form an important class of products and intermediates in the pharmaceutical, nutraceutical, cosmeceutical, agrochemical and flavour industries. For instance there are many biologically active compounds comprising such an arrangement of chiral atoms.

Asymmetric hydrogenation of prochiral olefins is an important technique in the synthesis of compounds having stereogenic centres on two adjacent carbon atoms. Homogeneous catalysts comprising chiral ligands, such as phosphate-ligands, chelating to a metal such as iridium, rhodium or ruthenium, are well known in the asymmetric hydrogenation of olefins. See for instance Handbook of Homogeneous Hydrogenation, de Vries and Elsevier (Eds.); Wiley-VCH, Weinheim, 2007.

Hydrogenation of a tetrasubstituted olefin potentially yields four different stereoisomeric products. Olefins where the two substituents on each of the two olefinic carbon atoms are different can have two stereoisomers, designated either E or Z, determined according to the Cahn-Ingold-Prelog priority system. Often, olefins exist as a mixture of both stereoisomers. For instance as the result of synthesis of an olefin both the E and Z stereoisomers may be formed. Typically, in a mixture of olefin steroisomers, one stereoisomer is present in a greater amount than the other. This can be due to factors such as steric (destabilizing) effects, or intramolecular hydrogen bonding (stabilising) effects, of one stereoisomer.

Further, it is known that photoirradiation of an olefin can lead to the conversion of one stereoisomer to the other, by a process known as isomerisation. Photoirradiation can lead to complete isomerisation of one stereoisomer to the other. Usually however this process leads to a mixture of E and Z olefins. Hydrogenation of each of the stereoisomers gives two different products. In general, four stereoisomeric products are possible by hydrogenation of a tetrasubstituted olefin. These can be defined from the absolute stereochemistry of the stereogenic centres as (R,R), (R,S), (S,R) and (S,S).

An isomerisation process is reported by Robinson et al, in J. Org. Chem. 66 (2001) 4148-4152, for the asymmetric synthesis of N,N'-protected 2,3-diaminobutanoic acid. Here, photoirradiation favours the Z-stereoisomer of the precursor olefin, whereas heating favours the E-stereoisomer. Pure E and pure Z stereoisomers were obtainable by employing one of the two techniques. Use of two different chiral catalysts allowed the authors to selectively obtain at will all four stereoisomers of the product.

Similarly, Elaridi et al, in Tetrahedron Asymmetry 16 (2005) 1309-1319, have shown that the isomerisation of ethyl 3-N-acetylamino-2-methyl-2-butenoate from the Z-stereoisomer to E-stereoisomer can be achieved in quantitative yields by photoirradiation. The E-stereoisomer is then hydrogenated to the desired corresponding butanoate by employing a stereoselective catalyst. This method gives access to all four stereoisomers of the butanoate, by employing two different catalysts to each of the two olefin stereoisomers.

In general, however, photoirradiation of a tetrasubstituted olefin does not give a substantially pure stereoisomer. In some cases an essentially equal proportion of each of the two stereoisomers is produced. Thus enantioselective hydrogenation of this mixture leads to the formation of two stereoisomeric products, one from the E-stereoisomer and one from the Z-stereoisomer.

The present inventors have however found a method which allows the asymmetric hydrogenation of a tetrasubstituted olefin to selectively yield any one of the four stereoisomers of the product compound (a compound having stereogenic centres on two adjacent carbon atoms) in high yield and high enantiomeric and diastereomeric excess. The method permits preferable asymmetric hydrogenation of one olefin stereoisomer in preference to the other, from a mixture of the two stereoisomers. This provides access to each of the two stereoisomeric products not obtainable from the other stereoisomer in high yield and with good stereoselectivity.

Accordingly the present invention provides a method for the preparation of a compound of formula (I), 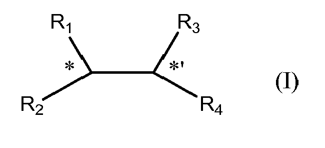 wherein R¹ is different to R²; R³ is different to R⁴; and R¹, R², R³ and R⁴ are each a moiety other than hydrogen, which method comprises,
i) photoirradiating at an intensity above ambient intensity a solution comprising a compound of formula (II), 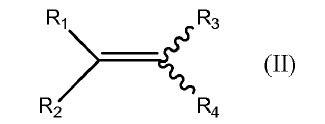 wherein R¹, R², R³ and R⁴ are as defined above, to form a solution of a mixture of compounds of formula (IIa) and (IIb), 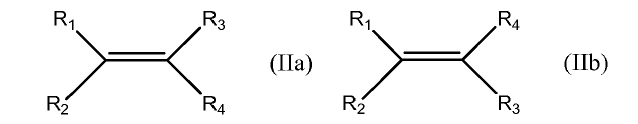 wherein R¹, R², R³ and R⁴ are as defined above; and
ii) contacting the mixture of compounds (IIa) and (IIb) with a hydrogen donating agent in the presence of an asymmetric hydrogenation catalyst, after initiation of i) and before termination of i).

The compound of formula (I) comprises two stereogenic centers, located on adjacent carbon atoms, denoted * and *'. The absolute configuration of each centre may be defined as either (R) or (S) according to the Cahn-Ingold-Prelog (CIP) system. Thus the compound of formula (I) may have the absolute configuration (R,R), (R,S), (S,R), or (R,S). The absolute configuration of a chiral centre of course depends on the specific substituents.

In principle each of R¹, R², R³ and R⁴ may be any moiety other than hydrogen. The combination of the four substituents must however form a compound of formula (II) (an olefin) sufficiently stable to undergo the specified reaction without the substituents themselves undergoing reaction to a significant extent.

The compound of formula (II) is either an E-stereoisomer or Z-stereoisomer. Preferably, the compound of formula (IIa) is the Z-stereoisomer of the compound of formula (II). Preferably, the compound of formula (IIb) is the E-stereoisomer of the compound of formula (II).

Photoirradiating means applying photoirradiation with a wavelength suitable to cause isomerisation of the olefin, and of sufficient intensity to cause isomerisation of the olefin. Photoirradiating may be carried out continuously or intermittently.

Typical wavelengths of photoirradiation are those corresponding to infrared, visible or ultraviolet (including far UV) radiation. Preferably the photoirradiation comprises a wavelength of from 100 to 500 nm; more preferably 200 to 400 nm. Suitable intensity of photoirradiation depends on the volume of the solution to be irradiated; defined herein as a volumetric intensity, in watts per litre (WL⁻¹). Typical volumetric intensity of the photoirradiation is from 10 to 1,000,000 WL⁻¹, measured as the total power of the irradiation source, for example the combined power rating of the lamps used. Preferred volumetric intensity is from 500 to 50,000 WL⁻¹; more preferably from 1,000 to 10,000 WL⁻¹

By an intensity above ambient intensity is meant photoirradiation is deliberately applied to the compound of formula (II) from a photoirradiation source. This specifically excludes ambient light from the sun or ambient room lighting, but includes strong sunlight deliberately applied to the solution either directly or by reflection. A suitable source of photoirradiation is, for example, a UV lamp, mercury lamp, (visible light) filament lamp, LED, broad spectrum lamp, laser or a fluorescent tube.

Photoirradiation is applied either continuously or intermittently. By continuously it is meant that photoirradiation is applied for a time period of exposure. By intermittently, it is meant photoirradiation is applied for a time period of exposure, then stopped for a time period of non-photoirradiation, then applied for at least one more time period of exposure. Therefore intermittent photoirradiation is analogous to alternating time periods of continuous photoirradiation and time periods of non-photoirradiation.

The time period of exposure of the solution may be different on each occurrence. The time period of exposure of the solution comprising a compound of formula (II) to photoirradiation on each occurrence, depends on a number of factors.
These include the nature of the compound of formula (II) itself, specifically how quickly it forms an equilibrium; the intensity and wavelength of applied photoirradiation; the nature of the solvent; concentration of the compound of formula (II); whether a photosensitiser is used; the dimensions of, and nature and thickness of the material of, a container holding the solution to be irradiated; the distance the solution is placed from the photoirradiation source; and whether the solution is circulated or stirred.

The necessary time period of exposure of both continuous and intermittent photoirradiation can be determined by routine experimentation by the person of skill in the art. The optimum time period of exposure for intermittent photoirradiation is that at which an equilibrium in the proportion of the two stereoisomers of the olefin is reached. A typical time period of exposure is up to 24 hours. Preferably the time period of exposure is from 3 minutes to 12 hours; more preferably from 5 minutes to 8 hours; more preferably from 10 minutes to 2 hours, most preferably from 1 to 1.5 hours. A typical time period of non-photoirradiation is up to 24 hours. Preferably the time period of non-photoirradiation is from 3 minutes to 12 hours; more preferably from 5 minutes to 8 hours; more preferably from 10 minutes to 2 hours, most preferably from 1 to 1.5 hours.

The optimum time period of exposure for continuous photoirradiation must take account of the rate of asymmetric hydrogenation.

Initiation of photoirradiation is the point in time at which photoirradiation is first applied to the solution, for example in intermittent photoirradiation the beginning of the first time period. Termination is the final point in time that photoirradiation is applied to the solution, for example in intermittent photoirradiation it is the end of the final time period of photoirradiation. Accordingly, initiation and termination of intermittent photoirradiation are separated by time periods of non-photoirradiation.

A hydrogen donating agent is contacted with the mixture of compounds (IIa) and (IIb) after initiation of photoirradiation and before termination of photoirradiation but may also be contacted with the mixture before initiation of photoirradiation and/or after termination of photoirradiation.

Olefins may undergo degradation under photoirradiation, particularly at high intensity, short or specific wavelengths, or long exposure times. For example, radicals may be generated, which give rise to side reactions such as rearrangement, dimerisation, polymerization or degradation. This may lead to reduced yield and problems with purification of the desired product. Use of one or more photosensitisers may help avoid such degradation by, for instance, reducing the time period of exposure to radiation required to achieve equilibrium of olefin stereoisomers, and/or absorbing photoirradiation at a wavelength which is not damaging to the olefin and then transferring the energy to the olefin to effect isomerisation.

A photosensitiser may therefore be present in at least i). A photosensitiser may be an aromatic hydrocarbon photosensitiser or an aromatic carbonyl photosensitiser. Examples of a suitable photosensitiser are toluene, phenol, acetone, benzonitrile, aniline, xanthone, anthrone, carbazole, benzophenone, triphenylene, phenanthrene, anthraquinone, naphthalene, 2-acetonaphthone, chrysene, 1-naphthaldehyde, benzil, acetophenone, 4-chloroacetophenone, 3-methoxyacetophenone, 4-methoxyacetophenone, 4-trifluoromethylacetophenone, 2,4-difluoropropiophenone, butyrophenone, benzylideneacetophenone and propiophenone. Acetone, propiophenone, carbazole, benzophenone, anthraquinone, acetophenone, 4-chloroacetophenone 2,4-difluoropropiophenone and butyrophenone are preferred. Propiophenone is more preferred for compounds of formula (II) in which R¹ is -NCH(O)R⁷ and R², R³ and R⁴ are each independently C₁-C₆ alkyl or aryl.

One or more filters may be placed between the source of radiation and the solution to be photoirradiated to block transmission of certain wavelengths. For example, short wavelengths may generate radicals causing the problems noted above. Accordingly filter(s) that block wavelengths below 300 nm or more preferably 350 nm are preferred. An example of a suitable filter is Pyrex®. One or more photosensitisers may be used in combination with the one or more filters.

The compound of formula (II) is present in a solution. Suitable solvents depend on the nature of the compound of formula (II) and the asymmetric hydrogenation catalyst. Typical solvents, especially suitable for the class of olefins exhibiting stabilization by intramolecular hydrogen bonding, are aprotic solvents, for example toluene, dichloromethane (DCM), tetrahydrofuran (THF), ethyl acetate, dioxane, acetone, and dimethyl carbonate. In the case of a compound of formula (II') apolar aprotic solvents, for example toluene or dichloromethane, are preferred. Other examples of solvents include protic solvents, for example water, methanol, ethanol and isopropanol. A mixture of two or more solvents may be used.

The asymmetric hydrogenation catalyst is any catalyst capable of asymmetrically hydrogenating a compound of formula (II). Selection of the most effective catalyst depends on the exact structure of the Compound of formula (II) to be hydrogenated. The solvent, concentration of compound of formula (II), pressure of hydrogen gas and reaction temperature may also affect the performance of the catalyst.

An asymmetric hydrogenation catalyst should preferably have high enantioselectivity. That is, for a specific stereoisomer of the olefin, the catalyst shows a strong preference, most preferably exclusivity, for hydrogenation to one of the two enantiomeric products. The catalyst typically shows a strong preference, most preferably exclusivity, for addition of hydrogen syn (or cis) across the C=C double bond.

An asymmetric hydrogenation catalyst should typically have high stereoselectivity. That is, the catalyst hydrogenates one of the olefin stereoisomers at a higher rate than the other olefin stereoisomer. This can be determined by measuring reaction rates of the two competing hydrogenation reactions. For example measurement of the rates of conversion may be executed by sampling the reaction at given time intervals and analyzing the samples by high performance liquid chromatography (HPLC), gas chromatography (GC) or nuclear magnetic resonance (NMR).

An asymmetric hydrogenation catalyst is selected to provide a high enantiomeric excess and a high diastereomeric excess. The choice of catalyst is made in order to optimize both these factors. The process of the present invention therefore provides high selectivity towards the product compound of formula (I). The catalyst can be selected by screening methods known in the art, for example by high throughput screening.

A typical asymmetric hydrogenation catalyst is a transition metal based catalyst. This can be prepared beforehand, and added as such, or it can be prepared in situ, i.e. by addition of a transition metal precursor and suitable ligands to the reaction vessel.

Suitable transition metal precursors are any available transition metal salt or complex. Preferably group VIII transition metal precursors are used, such as one derived from Ru, Ir or Rh. Suitable precursor examples are bis(1,5-cyclooctadiene)iridium tetrafluoroborate, bis[chloro-1,5-cyclooctadiene-iridium], bis(1,5-cyclooctadiene)-rhodium tetrafluoroborate, bis(2-methylallyl)(1,5-cyclooctadiene)ruthenium, dichlorobis[(p-cymene)chlororuthenium and dichloro(1,5-cyclooctadiene)ruthenium.

Suitable ligands are any compound with the possibility to donate electrons to the metal center, as known to the person skilled in the art, provided that the catalyst formed is an asymmetric hydrogenation catalyst. The ligands can be monodentate, bidentate, tridentate or tetradentate. More than one ligand can be used in combination with a transition metal ion. The ligands are preferably all chiral, but can alternatively be a mixture of non-chiral and chiral ligands. The amount of ligand with respect to the metal is not crucial. Preferably the amount of ligand is between 0.1 and 10, more preferably between 0.5 and 5 mol equivalents of the transition metal.

Suitable ligands are for example ligands described in Handbook of Homogeneous Hydrogenation, de Vries and Elsevier (Eds.); Wiley-VCH, Weinheim, 2007 or Comprehensive Asymmetric Catalysis I to III, Jacobsen, E., Pfaltz, A. and Yamamoto, H. (Eds.), Springer Verlag, 1999. Examples of types of suitable ligands are MonoPhos, DuPhos, JosiPhos, SolPhos, MandyPhos, TaniaPhos, WalPhos, PeaPhos and PipPhos. Specific examples include 3,3'-dimethyl MonoPhos, JosiPhos 13-1, JosiPhos 505-1, SolPhos SLA 001-1, MandyPhos SLM001, TaniaPhos SLT001-1, TaniaPhos SLT002-1, WalPhos W3-1 and PipPhos.

The catalyst is preferably used in quantities from 0.0001 to 10 mol-% based on the compound to be hydrogenated, the range 0.001 to 10 mol-% being especially preferred and the range 0.01 to 5 mol% being preferred in particular.

A hydrogen donating agent is a compound capable of donating a hydrogen atom to a compound of formula (II). As used herein the term hydrogen donating agent includes both compounds which transfer a hydrogen anion to the olefin, wherein the olefin subsequently bonds to a proton, for example from the solvent; and hydrogen gas (H₂), in which two atoms of hydrogen are added to the olefin. Examples of hydrogen donating compounds are isopropyl alcohol and hydrogen gas. Typically hydrogen gas is used as the hydrogen donating agent.

The process of the present invention may be carried out at low or elevated temperatures; typically at a temperature of from 0 to 80°C. Preferably, the temperature is from 10 to 60°C, more preferably from 15 to 50°C, for example 20 to 40°C.

The process of the present invention may be carried out at atmospheric pressure or at elevated pressure. In particular the hydrogen pressure, if hydrogen gas is used as a hydrogen donating agent, may be in the range of 0.1 to 200 bars of hydrogen. It is preferably present as a stoichiometric excess compared with the amount of the compound of formula (II). Preferably H₂ gas is supplied at a pressure of from 0.5 to 150 bar, more preferably from 1 to 100 bar, even more preferably 5 to 75 bar, most preferably 20 to 50 bar.

The compound of formula (II) is typically used in an amount of from 0.001 to 20 weight % of the solution to be irradiated. Preferably the compound of formula (II) is present in an amount of from 0.01 to 10 weight %; more preferably from 0.1 to 5 weight %.

Preferably one stereoisomer of the compound of formula (II) is produced by the photoirradiation in excess of the other stereoisomer. Most preferably one stereoisomer is present in only insubstantial amounts.

Photoirradiation causes isomerisation of the C=C double bond. This results in a shift in the amounts of the two olefin stereoisomers, the compounds of formula (IIa) and (IIb) (also known as E:Z (or Z:E) ratio). The nature of the olefin and the solvent determine where the point of equilibrium (prior to photoirradiation and after photoirradiation) lies. The conditions can be selected by routine experimentation to maximize this difference. By photoirradiating the mixture of stereoisomers it typically increases the amount of one stereoisomer as a proportion of the total amount of olefin steroeoisomers.

One stereoisomer of the olefin is typically hydrogenated by a given catalyst at a faster rate than the other stereoisomer. In one embodiment the catalyst is selected in order to hydrogenate the minor olefin steroisomer of the mixture prior to photoirradiation at a higher rate than the major stereoisomer prior to irradiation. This is because for a mixture of olefin stereoisomers the minor stereoisomer in a mixture is the more difficult to obtain without photoisomerisation of the olefin. Thus hydrogenation typically perturbs the equilibrium between the two olefin stereoisomers formed by photoirradiation.

In one embodiment the present invention provides a method as described above, which method comprises,
i) photoirradiating continuously at an intensity above ambient intensity a solution comprising a compound of formula (II), 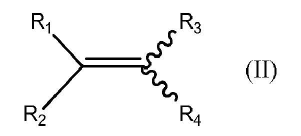 wherein R¹, R², R³ and R⁴ are as defined in claim 1, to form a solution of a mixture of compounds of formula (IIa) and (IIb), 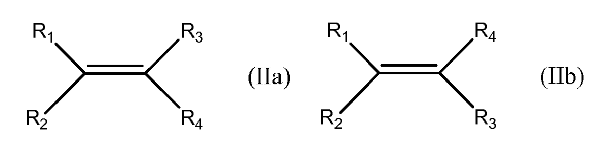 wherein R¹, R², R³ and R⁴ are as defined in claim 1; and simultaneously
ii) contacting the mixture of compounds (IIa) and (IIb) with a hydrogen donating agent in the presence of an asymmetric hydrogenation catalyst.

Simultaneously includes but is not limited to exclusively simultaneously. In other words during the process both photoirradiation and asymmetric hydrogenation will occur at the same time. This the case even though photoirradiation may be initiated before or after the supply of hydrogen donating agent is initiated, and the supply of hydrogen donating agent may be terminated before or after photoirradiation is terminated. Accordingly one embodiment of the present invention involves dynamic kinetic resolution.

In another embodiment the present invention provides a method as described above, which method comprises, in order,
i) photoirradiating at an intensity above ambient intensity a solution comprising a compound of formula (II), 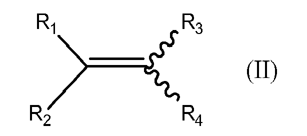 wherein R¹, R², R³ and R⁴ are as defined in claim 1, to form a solution of a mixture of compounds of formula (IIa) and (IIb), 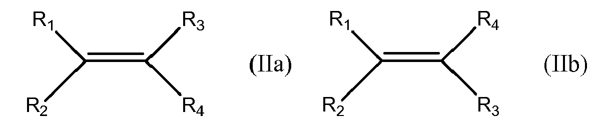 wherein R¹, R², R³ and R⁴ are as defined in claim 1;
ii) contacting the mixture of compounds of formula (IIa) and (IIb) with a hydrogen donating agent in the presence of an asymmetric hydrogenation catalyst; and
iii) repeating at least once i) and ii) in order.

Steps i) and ii) are carried out, in order, at least twice. Typically they are carried out between two and six times. Preferably, they are carried out two times, three times or four times. More preferably the steps i) and ii) are carried out, in order, two times or most preferably three times. Preferably steps i) and ii) are carried out exclusively of each other. For example, steps i) and ii) are carried out such that i) ends, ii) starts then ends, then i) starts again. The number of times i) and ii) are repeated depends on the rate difference of hydrogenation of the E and Z stereoisomers compound of formula (II); and the ratio of E and Z stereoisomers prior to photoirradiation compared with the ratio of E and Z stereoisomers following photoirradiation.

Preferably i) and ii) are carried out in the same reaction vessel.
Accordingly at least a portion of the reaction vessel must be of sufficient transparency to the photoirradiation applied.

Reiteration or continued application of the photoirradiation acts to restore the equilibrium between the two olefin stereoisomers. In this way a solution of the compound of formula (II) where photoirradiation yields low, or even no, isomeric excess of one olefin stereoisomer over the other, may produce one hydrogenated product stereoisomer of the compound of formula (I) in a proportional yield higher than the initial E:Z ratio of the compound of formula (II).

Accordingly, in one embodiment the present invention makes use of an iterative process to produce the desired product. It is possible to obtain high yields of the desired product using the iterative method of this embodiment of the present invention. This is sometimes termed a "pseudo-dynamic kinetic resolution" or "pseudo-dynamic kinetic asymmetric transformation".

The present invention is particularly useful when the compound of formula (I) is difficult to obtain by other methods. Thus it becomes more useful when, in the solution prior to photoirradiating, the ratio of one olefin stereoisomer to the other olefin stereoisomer is high, for example 70:30, 80:20, 90:10 or 95:5. Similarly, the method becomes more useful when, in the solution after photoirradiation, the ratio of one olefin stereoisomer to the other olefin stereoisomer is low, for example 30:70, 20:80, 10:90 or 5:95.
Figure 1 shows an example reaction scheme of an embodiment of the present invention, involving an iterative process (pseudo-dynamic kinetic resolution).
Figure 2 shows an example reaction scheme of an embodiment of the present invention, involving a dynamic kinetic resolution.
Figure 3 shows the % conversion of a compound of formula (II) into a compound of formula (I) by an example of the present invention which is an iterative process.

Typically R¹, R², R³ and R⁴ are each independently C₁-C₁₂ alkyl, C₂-C₁₂ alkenyl, C₂-C₁₂ alkynyl, C₁-C₁₂ alkoxy, aryl, aryloxy, heteroaryl, carbocyclyl, heterocyclyl, - C(O)R⁵, -NR⁶₂, -SR^{8a}, -S(O)R^{8b}, -SO₂R^{8b}, -OR⁹, -NO₂ or -CN, wherein,

R⁵ is hydrogen, hydroxy, -O⁻, -O⁻Q⁺, C₁-C₁₂ alkyl, C₁-C₁₂ alkoxy, C₂-C₁₂ alkenyl, C₂-C₁₂ alkynyl, aryl, aryloxy, heteroaryl, carbocyclyl, heterocyclyl, -NR¹⁰₂ or -SR¹¹;
each R⁶ is independently hydrogen, C₁-C₁₂ alkyl, C₂-C₁₂ alkenyl, C₂-C₁₂ alkynyl, aryl, heteroaryl, carbocyclyl, heterocyclyl or -C(O)R⁷, or both R⁶ together with the nitrogen to which they are attached form a nitrogen-containing heterocyclyl or nitrogen-containing heteroaryl group;
R⁷ is hydrogen, C₁-C₁₂ alkyl, C₁-C₁₂ alkoxy, C₂-C₁₂ alkenyl, C₂-C₁₂ alkynyl, aryl, aryloxy, heteroaryl, carbocyclyl or heterocyclyl;
R^{8a} is hydrogen, C₁-C₁₂ alkyl, C₂-C₁₂ alkenyl, C₂-C₁₂ alkynyl, aryl, heteroaryl, carbocyclyl or heterocyclyl;
R^{8b} is hydroxy, -O⁻, -O⁻Q⁺, C₁-C₁₂ alkyl, C₂-C₁₂ alkenyl, C₂-C₁₂ alkynyl, aryl, heteroaryl, carbocyclyl or heterocyclyl;
R⁹ is C₁-C₁₂ alkyl, C₂-C₁₂ alkenyl, C₂-C₁₂ alkynyl, aryl, heteroaryl, carbocyclyl, heterocyclyl or -SiR¹²₃;
each R¹⁰ is independently hydrogen, C₁-C₁₂ alkyl, C₂-C₁₂ alkenyl, C₂-C₁₂ alkynyl, aryl, heteroaryl, carbocyclyl or heterocyclyl, or both R¹⁰ together with the nitrogen to which they are attached form a nitrogen-containing heterocyclyl or nitrogen-containing heteroaryl group;
R¹¹ is hydrogen, C₁-C₁₂ alkyl or aryl;
each R¹² is independently hydrogen, C₁-C₁₂ alkyl or aryl; and
each Q⁺ is independently an alkali metal ion or an ammonium ion.

Preferably, R¹ is -NR⁶₂. More preferably R¹ is -NHR⁶, -NR⁶C(O)R⁷. Typically, when R¹ is -NR⁶C(O)R⁷, R⁶ is hydrogen. Typically, when R¹ is -NR⁶C(O)R⁷, R⁷ is C₁-C₆ alkyl, C₁-C₆ alkoxy, aryl or aryloxy, preferably, C₁-C₆ alkyl or aryl.

Preferably R² is C₁-C₆ alkyl, aryl, heteroaryl, carbocyclyl or heterocyclyl. More preferably, R² is C₁-C₆ alkyl, or aryl.

There may be a stabilising intramolecular interaction between the group R¹ and R³. An example of a stabilising intramolecular interaction is intramolecular hydrogen bonding. For instance R³ may contain an atom such as oxygen or nitrogen able to form a hydrogen bond with a hydrogen atom bonded to an oxygen or nitrogen of R¹.

Preferably R³ is a group which is capable of forming a stabilizing intramolecular interaction with R¹. More preferably R³ is -C(O)R⁵, -S(O)R^{8b} or -SO₂R^{8b}. Still more preferably R³ is -C(O)R⁵. When R³ is -C(O)R⁵, typically R⁵ is C₁-C₆ alkyl, C₁-C₆ alkoxy, aryl or aryloxy, preferably C₁-C₆ alkyloxy. Most preferably R³ is -C(O)-C₁-C₆ alkoxy.

Preferably R⁴ is C₁-C₆ alkyl, aryl, heteroaryl, carbocyclyl, or heterocyclyl. More preferably, R⁴ is C₁-C₆ alkyl or aryl.

Preferably R⁵ is hydroxy, -O⁻, -O⁻Q⁺, C₁-C₆ alkyl, C₁-C₆ alkoxy, C₂-C₆ alkenyl, C₂-C₆ alkynyl, aryl, aryloxy, heteroaryl, carbocyclyl, heterocyclyl or -NR¹⁰₂. More preferably R⁵ is hydroxy, -O⁻, -O⁻Q⁺, C₁-C₆ alkyl, C₁-C₆ alkoxy, aryl or aryloxy. More preferably R⁵ is hydroxy, -O⁻, -O⁻Q⁺, C₁-C₆ alkoxy or aryloxy. Most preferably R⁵ is C₁-C₆ alkoxy.

Preferably R⁶ is hydrogen, C₁-C₆ alkyl, aryl, heteroaryl, carbocyclyl, heterocyclyl or -C(O)R⁷. More preferably R⁶ is hydrogen, C₁-C₆ alkyl, aryl or -C(O)R⁷. When R⁶ is -C(O)R⁷, preferably R⁷ is C₁-C₆ alkyl or aryl. Most preferably at least one R⁶ is hydrogen.

Preferably R⁷ is hydrogen, C₁-C₆ alkyl, aryl or aryloxy. More preferably R⁷ is C₁-C₆ alkyl or aryl.

Preferably R^{8a} is C₁-C₆ alkyl or aryl; more preferably C₁-C₆ alkyl.

Preferably R^{8b} is C₁-C₆ alkyl, C₁-C₆ alkoxy or aryl; more preferably C₁-C₆ alkyl or C₁-C₆ alkoxy.

Preferably R⁹ is C₁-C₆ alkyl, aryl or -SiR¹²₃; more preferably C₁-C₆ alkyl or -SiR¹²₃.

Preferably, R¹⁰ is hydrogen, C₁-C₆ alkyl, aryl, heteroaryl, carbocyclyl or heterocyclyl. More preferably R¹⁰ is hydrogen, C₁-C₆ alkyl or aryl. Most preferably at least one R¹⁰ is hydrogen.

Preferably R¹¹ is hydrogen, C₁-C₁₂ alkyl or aryl.

Preferably two of the three R¹² on a given Si atom are C₁-C₆ alkyl or aryl, and the third is hydrogen, C₁-C₆ alkyl or aryl.

Preferably Q⁺ is a sodium, potassium or lithium ion, or a trialkyl ammonium or tetraalkyl ammonium ion.

In a preferred embodiment of the present invention,
R¹ is -NR⁶C(O)R⁷;
R² is C₁-C₆ alkyl, aryl, heteroaryl, carbocyclyl or heterocyclyl;
R³ is -C(O)R⁵, -S(O)R^{8b}, -SO₂R^{8b}, C₁-C₆ alkyl or aryl;
R⁴ is C₁-C₆ alkyl, aryl, heteroaryl, carbocyclyl or heterocyclyl;
R⁵ is hydroxy, -O⁻, -O⁻Q⁺, C₁-C₆ alkyl, C₁-C₆ alkoxy, C₂-C₆ alkenyl, C₂-C₆ alkynyl, aryl, aryloxy, heteroaryl, carbocyclyl, heterocyclyl or -NR¹⁰₂;
R⁶ is hydrogen, C₁-C₆ alkyl, aryl, heteroaryl, carbocyclyl or heterocyclyl;
R⁷ is hydrogen, C₁-C₆ alkyl, C₁-C₆ alkoxy, aryl, aryloxy, heteroaryl, carbocyclyl or heterocyclyl;
R^{8b} is C₁-C₆ alkyl or C₁-C₆ alkoxy;
R¹⁰ is hydrogen, C₁-C₆ alkyl, aryl, heteroaryl, carbocyclyl or heterocyclyl; and
Q⁺ is a sodium, potassium or lithium ion, or a trialkyl ammonium or tetraalkyl ammonium ion.

In a particularly preferred embodiment of the present invention, R¹ is - NR⁶C(O)R⁷; R³ is -C(O)-O-C₁-C₆ alkyl; and each of R² and R⁴ is independently C₁-C₆ alkyl, aryl, carbocyclyl or heterocyclyl. More preferably R¹ is -NHC(O)OR⁷, where R⁷ is C₁-C₆ alkyl or aryl; R³ is -C(O)-C₁-C₆ alkyl; and each of R² and R⁴ is independently C₁-C₆ alkyl, or aryl.

In another embodiment of the present invention, R¹ is -NHC(O)R⁷; and R², R³ and R⁴ are each independently C₁-C₆ alkyl or aryl. Preferably R⁷ is C₁-C₆ alkyl or aryl.

As used herein C₁-C₁₂ alkyl means an alkyl group comprising from 1 to 12 carbon atoms. It may be branched or unbranched. Typically it is a C₁-C₆ alkyl group, preferably a C₁-C₄ alkyl group. Examples include methyl, ethyl, n-propyl, i-propyl, n-butyl, s-butyl and t-butyl. Unless otherwise specified, an alkyl group may be substituted or unsubstituted. A preferred substituent on a substituted C₁-C₁₂ alkyl group is halogen, C₁-C₆ alkoxy, aryl, aryloxy, heteroaryl, carbocyclyl or heterocyclyl.

As used herein C₂-C₁₂ alkenyl means an alkyl group comprising from 2 to 12 carbon atoms and at least one C=C bond. It may be branched or unbranched. Typically it is a C₂-C₆ alkenyl group, preferably C₂-C₄ alkenyl. Examples include ethenyl, propenyl and butenyl. Unless otherwise specified, an alkenyl group may be substituted or unsubstituted. A preferred substituent on a substituted C₂-C₁₂ alkenyl group is C₁-C₆ alkoxy, aryl, aryloxy, or heteroaryl.

As used herein C₂-C₁₂ alkynyl means an alkyl group comprising from 2 to 12 carbon atoms and at least one C≡C bond. It may be branched or unbranched. Typically it is a C₂-C₆ alkynyl group. Preferably it is a C₂-C₄ alkynyl group. An example is ethynyl (acetylenyl). Unless otherwise specified, an alkynyl group may be substituted or unsubstituted. A preferred substituent on a substituted C₂-C₁₂ alkynyl group is C₁-C₆ alkoxy, aryl, aryloxy, or heteroaryl.

As used herein C₁-C₁₂ alkoxy means a C₁-C₁₂ alkyl group as defined above bonded through an oxygen atom. Similarly a C₁-C₆ alkoxy group is a C₁-C₆ alkyl group as defined above bonded through an oxygen atom.

As used herein C₁-C₆ haloalkyl means a C₁-C₆ alkyl group substituted by at least one halogen. Typically it is substituted by 1, 2 or 3 halogen atoms, preferably 1 halogen. Preferred halogens are chlorine and bromine. Typically a C₁-C₆ haloalkyl is a C₁-C₄ haloalkyl, for example methyl chloride, dimethyl chloride, trimethylchloride, methyl bromide, demithylbromide, trimethyl bromide, ethyl chloride or ethyl bromide.

As used herein aryl means a mono-, bi- or tricyclic aromatic hydrocarbon ring system bonded through an aromatic carbon atom. Aryl includes fused ring systems in which an aryl group is fused to a monocyclic carbocyclyl or monocyclic heterocyclyl group. Examples of aryl groups include phenyl, naphthyl and anthracyl groups. Phenyl is preferred. Unless otherwise specified, an aryl group may be substituted or unsubstituted. Preferred substituents on an aryl group are C₁-C₆ alkyl, C₁-C₆ haloalkyl, C₁-C₆ alkoxy, aryloxy, halogen and nitro.

As used herein aryloxy means an aryl group as defined above bonded through an oxygen atom.

As used herein heteroaryl means a mono-, bi- or tricyclic aromatic ring system wherein at least one ring contains at least one heteroatom selected from O, N and S. A nitrogen-containing heteroaryl is a heteroaryl which comprises at least one nitrogen atom in the ring. Heteroaryl includes fused ring systems in which a heteroaryl group is fused to a monocyclic carbocyclyl or heterocyclyl group. Typically it contains one or two heteroatoms, preferably one, in any one aromatic ring. Preferably it is a monocyclic system. Examples include pyridyl, pyrazinyl, pyridazinyl, pyrimidinyl, pyrrolyl, furanyl, thienyl, imidazolyl, pyrazolyl, oxazole, isoxazolyl, thiazolyl and isothiazolyl. Unless otherwise specified, a heteroaryl group may be substituted or unsubstituted, but is preferably unsubstituted.

As used herein, a carbocyclyl group is a non-aromatic saturated or unsaturated hydrocarbon ring. Typically it has from 3 to 7 carbon atoms. Preferably it is a saturated hydrocarbon ring (i.e. a cycloalkyl group). Preferably it has 5 or 6 carbon atoms. Examples include cyclopropyl, cyclobutyl, cyclopentyl and cyclohexyl. It is preferably cyclopentyl or cyclohexyl. Unless otherwise specified, a carbocyclyl group may be substituted or unsubstituted, but is preferably unsubstituted.

As used herein, heterocyclyl means a non-aromatic, mono-, bi-, or tricyclic saturated or unsaturated carbocyclic ring, typically having from 5 to 10 carbon atoms, in which one or more, for example 1, 2 or 3, of the carbon atoms is replaced by a heteroatom selected from N, O and S. A nitrogen-containing heterocyclyl is a heterocyclyl which comprises at least one nitrogen atom in the ring. Saturated heterocyclyl groups are preferred. Examples include tetrahydrofuranyl, tetrahydrothienyl, pyrrolidinyl, imidazolidinyl, pyrazolidinyl, dioxolanyl, thiazolidinyl, tetrahydropyranyl, piperidinyl, dioxanyl, piperazinyl, morpholinyl, thiomorpholinyl and thioxanyl. Preferably heterocyclyl is dioxolanyl or 1,3-dioxanyl. Unless otherwise specified, a heterocyclyl group may be substituted or unsubstituted, but is preferably unsubstituted.

As used herein, nitro means a moiety -NO₂.

As used herein, a halogen is typically chlorine, fluorine, bromine or iodine and is preferably bromine, chlorine or fluorine, most preferably chlorine.

A group is substituted when one or more hydrogen atoms on the group is replaced by a different moiety. The number of carbon atoms of a substituent is not included in the number of carbon atoms of the group to which it is a substituent. For example benzyl is defined as C₁ alkyl substituted by phenyl, not as C₇ alkyl substituted by phenyl. Where substituted, typically a group bears one or two substituents, preferably one. A substituent is itself unsubstituted or substituted by halogen, unsubstituted C₁-C₆ alkyl, unsubstituted C₁-C₆ alkoxy or C₁-C₆ haloalkyl. Typical substituents are C₁-C₆ alkyl, C₂-C₆ alkenyl, C₂-C₆ alkynyl, C₁-C₆ alkoxy, aryl, aryloxy, heteroaryl, carbocyclyl, heterocyclyl, -C(O)R^{5'}, -NR^{6'}₂, -SR^{8'}, -S(O)R^{8'}, -S(O)₂R^{8'}, -OR^{9'}, nitro or halogen, wherein,
R^{5'} is hydrogen, hydroxy, C₁-C₆ alkyl, C₁-C₆ alkoxy, C₂-C₆ alkenyl, C₂-C₆ alkynyl, aryl, aryloxy, heteroaryl, carbocyclyl, heterocyclyl, -NR^{10'}₂ or halogen;
each R^{6'} is hydrogen, C₁-C₆ alkyl, C₂-C₆ alkenyl, C₂C₆ alkynyl, aryl, heteroaryl, carbocyclyl, heterocyclyl or -C(O)R^{7'};
R^{7'} is hydrogen, C₁-C₆ alkyl, aryl or aryloxy;
R^{8'} is C₁-C₆ alkyl;
R^{9'} is C₁-C₆ alkyl; and

Each R^{10'} is hydrogen, C₁-C₆ alkyl, C₂-C₆ alkenyl, C₂C₆ alkynyl, aryl, heteroaryl, carbocyclyl or heterocyclyl.

Preferred substituents are in general unsubstituted C₁-C₆ alkyl, unsubstituted C₁-C₆ haloalkyl, unsubstituted C₁-C₆ alkoxy, aryl, aryloxy, heterocyclyl, hydroxy, halogen or -C(O)R, wherein R is unsubstituted C₁-C₆ alkoxy or unsubstituted aryloxy, and wherein the aryl, heteroaryl, carbocylcyl, heterocyclyl are either unsubstituted or are themselves substituted by halogen, unsubstituted C₁-C₆ alkyl, unsubstituted C₁-C₆ haloalkyl, unsubstituted C₁-C₆ alkoxy or hydroxy.

More preferred substituents are unsubstituted C₁-C₄ alkyl, unsubstituted C₁-C₄ haloalkyl, unsubstituted C₁-C₄ alkoxy, phenyl, hydroxy, halogen, heterocyclyl, and - C(O)R', where R' is hydrogen, unsubstituted C₁-C₄ alkyl, C₁-C₄ alkoxy or hydroxy, and wherein the phenyl and heterocyclyl are each independently either unsubstituted or substituted by halogen, unsubstituted C₁-C₄ alkyl, unsubstituted C₁-C₄ haloalkyl or unsubstituted C₁-C₄ alkoxy. Typically heterocyclyl is dioxolanyl or 1,3-dioxanyl.

Formula (I) encompasses the following four stereoisomers, (Ia), (Ib), (Ic) and (Id), wherein, R¹ to R⁴ are as defined above.

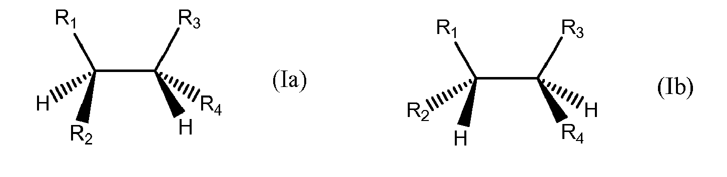

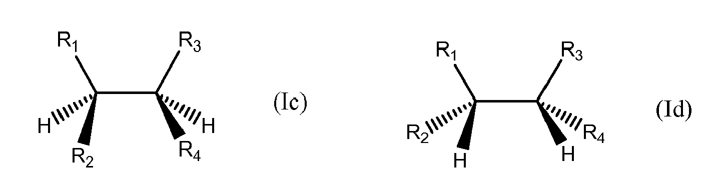

(Ia) and (Ib) are preferentially formed on hydrogenation of (IIb). Similarly, (Ic) and (Id) are preferentially formed on hydrogenation of (IIa). However, in the case where =C(R¹)(R²) is identical to =C(R³)(R⁴) only 3 stereoisomeric product compounds of formula (I) are formed: two diastereomers and a meso compound. For example, if R¹ is identical to R³ and R² is identical to R⁴, then (Ic) will be identical to (Id). The skilled person would understand therefore that not every compound of formula (II) has four potential products of formula (I).

One embodiment of the present invention comprises photoirradiation of a solution comprising a compound of formula (IIa) to yield a stereoisomeric mixture of (IIa) and (IIb), wherein (IIb) is present in excess over (IIa), followed by asymmetric hydrogenation, to yield a compound of formula (Ia) or (Ib). The compound of formula (Ia) may be preferentially produced by use of an asymmetric hydrogenation catalyst that has a high enantioselectivity for producing that compound over the compound of formula (Ib). Equally, the compound of formula (Ib) may be preferentially produced by use of an asymmetric hydrogenation catalyst that has a high enantioselectivity for producing that compound over the compound of formula (Ia). An appropriate catalyst can be selected by routine experimentation by the person of skill in the art.

Another embodiment of the present invention comprises photoirradiating a solution comprising a compound of formula (IIb) to yield a stereoisomeric mixture of (IIa) and (IIb), wherein (IIa) is present in excess over (IIb), followed by asymmetric hydrogenation, to yield a compound of formula (Ic) or (Id). The compound of formula (Ic) may be preferentially produced by use of an asymmetric hydrogenation catalyst that has a high enantioselectivity for producing that compound over the compound of formula (Id). Equally, the compound of formula (Id) may be preferentially produced by use of an asymmetric hydrogenation catalyst that has a high enantioselectivity for producing that compound over the compound of formula (Ic). An appropriate catalyst can be selected by routine experimentation by the person of skill in the art.

Preferably, in the method of the present invention, the compound of formula (II) is a compound of formula (IIa), 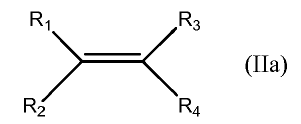 wherein R¹, R², R³ and R⁴ are as defined in claim 1,
and the compound of formula (I) is a compound of formula (Ia) or a compound of formula (Ib), 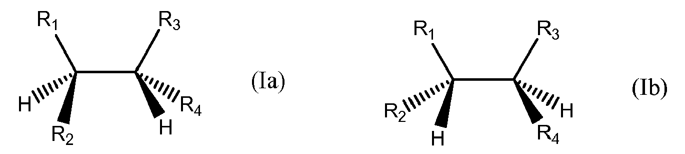 wherein R¹, R², R³ and R⁴ are as defined above.

Preferably, in the method of the present invention, the compound of formula (I) is a compound of formula (I'), the compound of formula (II) is a compound of formula (II'), the compound of formula (IIa) is a compound of formula (IIa'), and the compound of formula (IIb) is a compound of formula (IIb'), 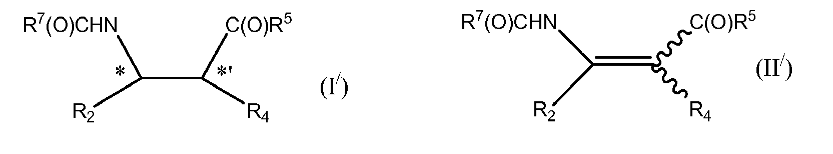 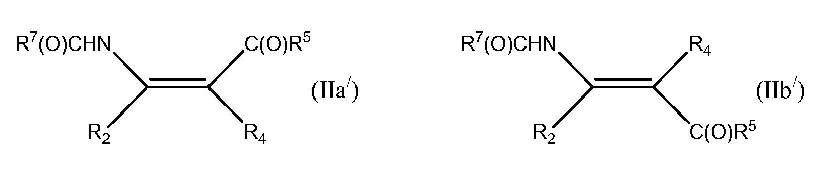 wherein,
R² and R⁴, are each as defined above;
R⁵ is hydroxy, -O⁻, -O⁻Q⁺, C₁-C₆ alkyl, C₁-C₆ alkoxy, C₂-C₆ alkenyl, C₂-C₆ alkynyl, aryl, aryloxy, heteroaryl, carbocyclyl, heterocyclyl or -NR¹⁰₂;
R⁷ is hydrogen, C₁-C₆ alkyl, C₁-C₆ alkoxy, aryl, aryloxy, heteroaryl, carbocyclyl or heterocyclyl;
R¹⁰ is hydrogen, C₁-C₆ alkyl, aryl, heteroaryl, carbocyclyl or heterocyclyl; and
Q⁺ is a sodium, potassium or lithium ion, or a trialkyl ammonium or tetraalkyl ammonium ion; except that,
R² is not NHC(O)R⁷, and R⁴ is not C(O)R⁵.

In one embodiment the present invention provides a method as described above, wherein the compound of formula (I) is a compound of formula (I"), the compound of formula (II) is a compound of formula (II"), the compound of formula (IIa) is a compound of formula (IIa"), and the compound of formula (IIb) is a compound of formula (IIb"), 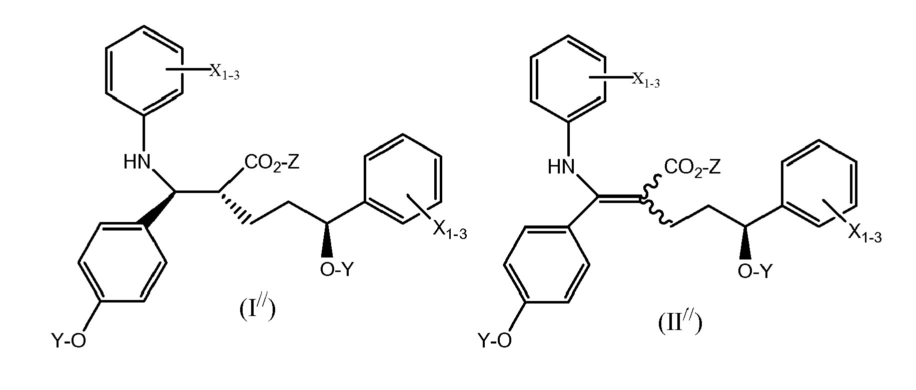 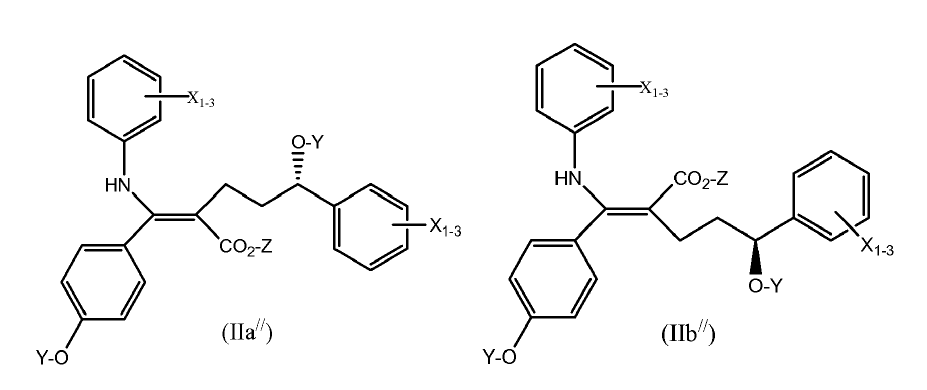 wherein X is a halogen, and each of Y and Z is a protecting group.

X is typically fluorine. Preferably only one X is present on each phenyl ring; most preferably in the 4-position.

Y is typically C₁-C₆ alkyl or aryl. Preferably Y is benzyl.

Z is typically C₁-C₆ alkyl or aryl. Preferably Z is C₁-C₄ alkyl; more preferably ethyl.

Preferably, the method further comprises cyclising the compound of formula (I*^{II}*) to form a compound of formula (III), 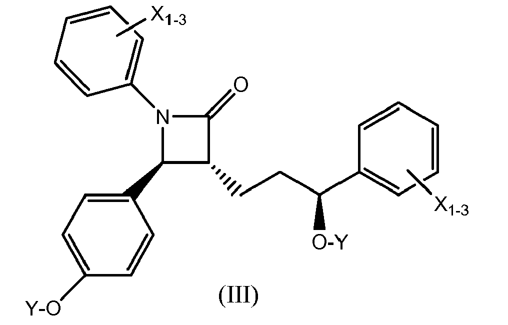 and optionally removing the protecting groups from the compound of formula (III) to form a compound of formula (IV), 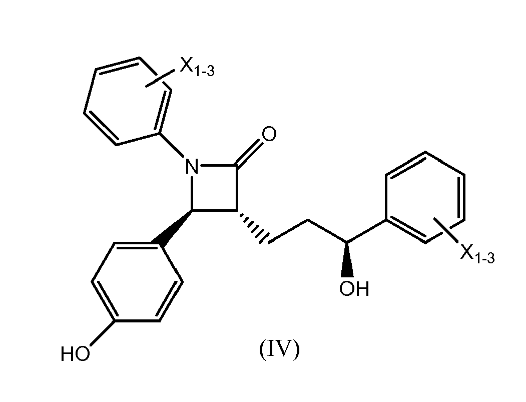 wherein X is as defined above.

Cyclising the compound of formula (I*^{II}*) to form a compound of formula (III) is achieved by techniques known to the person of skill in the art.

Typically, the compound of formula (IV) is produced and said compound of formula (IV) is (3R,4S)-1-(4-fluorophenyl)-3-[(3S)-3-(4-fluorophenyl)-3-hydroxypropyl]-4-(4-hydroxyphenyl)azetidin-2-one. This compound itself is also known as ezetimibe, and is depicted:

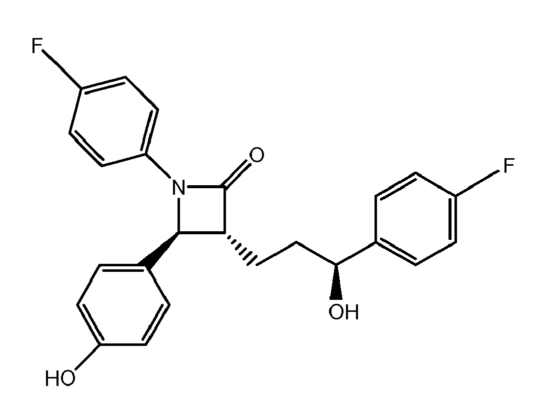

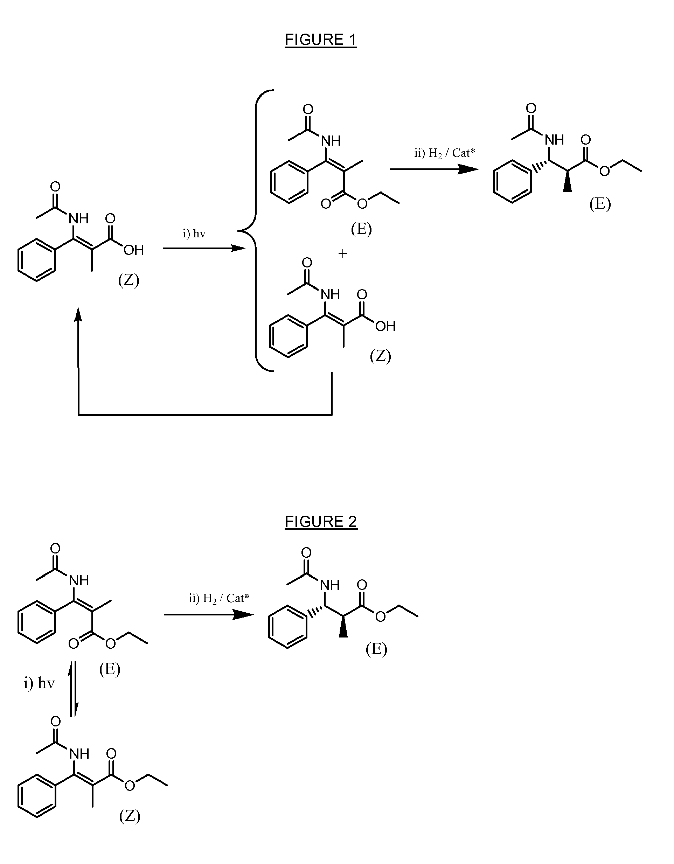

Figure 1 shows an example reaction scheme of an embodiment of the present invention, involving a pseudo-dynamic kinetic resolution. Step i) involves photoirradiation of the Z-stereoisomer of an enamide to give a mixture of E and Z stereoisomers. Step ii) involves the hydrogenation of the resulting mixture of Z and E stereoisomers of the compound of formula (II), in the presence of an asymmetric hydrogenation catalyst which is selective for the E-stereoisomer: effectively only the E-stereoisomer of the enamide is hydrogenated to essentially one of the enantiomers. The Z-stereoisomer remains part of the mixture of enamide stereoisomers which is photoirradiated in a further iteration of step i) to form an equilibrium.

Figure 2 shows an example reaction scheme of an embodiment of the present invention, involving a dynamic kinetic resolution. Steps i) and ii) are carried out simultaneously. The Z-stereoisomer of the enamide is photoirradiated to produce a mixture of E and Z stereoisomers. The E-stereoisomer is selectively hydrogenated using an asymmetric hydrogenation catalyst which is selective for the E-stereoisomer. Accordingly the equilibrium of enamide stereoisomers is driven towards the E-steroisomer. The E-stereoisomer is hydrogenated to form a compound of formula (I) at a fast rate. The Z-stereoisomer is hydrogenated at a very slow rate and, as illustrated, the hydrogenated product of the Z-stereoisomer is not produced because the E:Z equilibrium is adjusted by the (simultaneous) photoirradiation in step i).

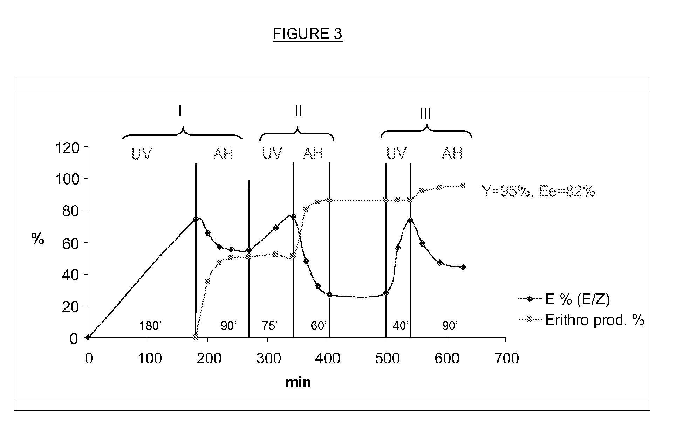

Figure 3 shows the % conversion of a compound of formula (II) into a compound of formula (I) by an example of the present invention which is an iterative process. The data is taken from Example 11. The x axis is time, and the y axis % yield of desired erythroenantiomer and proportion of E-stereoisomer out of total amount of enamide remaining.. UV is the time period during which ultraviolet photoirradiation is performed and AH is the time period during which asymmetric hydrogenation is performed. I, II and III each indicate one iteration of the process of UV followed by AH. The line defined by diamonds is the % of E-stereoisomer in the mixture of E and Z enamide stereoisomers of formula (II) (E-3 and Z-3). The line defined by squares is the % yield of the desired compound of formula (I) (Erithro-18) based on the compound of formula (II) (Z-3). Ee is the enantiomeric excess of Erithro-18. Y is the final yield of Erithro-13.

The present invention is illustrated by the following examples but is not intended to be limited thereto.

In the following examples, unless stated otherwise, chemicals were obtained from commercial sources and used without further purification. ¹H- and ¹³C-NMR spectra were recorded on a Bruker Avance 300 MHz NMR (75.48 MHz) spectrometer. Chemical shift values are denoted in δ values (ppm) relative to residual solvent peaks (CDCl₃ ¹H δ = 7.26, ¹³C δ = 76.9; CD₃OD ¹H δ = 3.31, ¹³C δ = 49.0).

Column chromatography was performed using silica gel, Merck type 9385 230-400 mesh. Thin layer chromatography (TLC) was performed on pre-coated silica gel 60 F₂₅₄ plates (Merck); spots were visualized using a) UV light or b) KMnO₄ or c) a mixture of phosphomolybdic acid (25 g), cerium (IV) sulfate (7.5 g), water (500 mL) and H₂SO₄ (25 ml) or d) a mixture of p-methoxybenzaldehyde (2.1 mL), AcOH (1.8 mL), H₂SO₄ (6.3 mL) and EtOH (170 mL).

UV-light experiments were conducted using one of the following reactors:
A) Rayonet photochemical chamber reactor model RPR-200 containing 16 UV-lamps. Two different sets of lamps were used: 1) 16 lamps (21 watts each) with a maximum intensity at 300 nm (range 250-350 nm); 2) 16 lamps (24 watts each) with a maximum intensity at 350 nm (range 300-400 nm).
B) Peschl UV-reactor system containing a TQ 150 medium pressure mercury lamp (150 watts lamp with a range from 180 to 600 nm and maxima at 254, 313, 366, 546 and 577/79 nm).

Analytical HPLC diagrams were recorded on an HP1090 Liquid Chromatograph. UV detection was performed at 220 nm using a UV-VIS 204 Linear spectrometer.

### Preparation Example 1

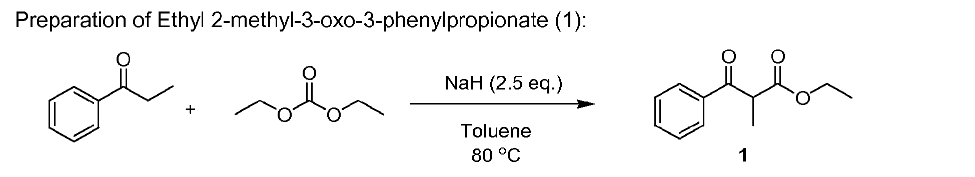

Sodium hydride (60% w/w, 10.0 g, 250 mmol, 2.5 eq.) was suspended in dry toluene (50 mL) and diethyl carbonate (24.4 mL, 200 mmol, 2.0 eq.) was added. The resulting suspension was heated to 80 °C and subsequently propiophenone (13.4 mL, 100 mmol, 1.0 eq.) was added over 1 hour using a syringe pump. The mixture was cooled to room temperature and then poured into ice-water (150 mL) containing glacial acetic acid (25 mL). The organic layer was separated and the aqueous layer was extracted with MTBE (200 mL). The combined organic layers were dried (Na₂SO₄), filtered and concentrated in vacuo to give the desired β-ketoester 1 as a yellowish liquid. The product was used in the next step without further purification.

¹H-NMR of 1 (CDCl₃, 300 MHz) δ = 1.17 (t, 3H, J = 7.2 Hz), 1.50 (d, 3H, J = 6.9 Hz), 4.18 (q, 2H, J = 7.2 Hz), 4.38 (q, 1H, J = 6.9 Hz), 7.48 (m, 2H), 7.58 (m, 1H), 7.99 (d, 2H, J = 7.2 Hz) ppm.

### Preparation Example 2

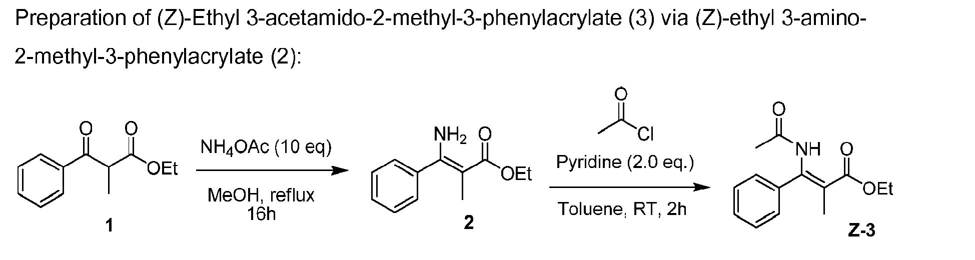

β-ketoester 1 (20.6 g, 100 mmol) and ammonium acetate (77 g, 1.0 mol, 10.0 eq.) were dissolved in MeOH (350 mL) and the resulting mixture was heated under refluxing conditions for 16 hours while stirring under a nitrogen atmosphere (GC showed approximately 88% conversion). The reaction mixture was concentrated in vacuo and the product was diluted with water (200 mL) and EtOAc (200 mL). The aqueous layer was extracted with EtOAc (200 mL) and the combined organic layers were dried (Na₂SO₄) filtered and concentrated in vacuo to give a mixture of β-ketoester 1 (12%) and enamine 2 (88%) as a yellow/brown oil. The product was used in the next step without purification.

Enamine 2 (88 mmol) was dissolved in toluene (400 mL) and pyridine (19.8 mL, 200 mmol, 2.27 eq.) and acetyl chloride (7.90 mL, 110 mmol, 1.25 eq.) were added. The resulting solution was stirred under a nitrogen atmosphere for 3 hours and then quenched by addition of HCl (2M aq., 100 mL). The organic layer was washed with HCl (2M aq., 200 mL) and water (200 mL), dried (Na₂SO₄), filtered and concentrated in vacuo. The product was purified by column chromatography (n-heptane-EtOAc 3:1 to 2:1 v/v) to give Z-3 (16 g, 65 mmol, 65% over 3 steps) as an off-white solid.

¹H-NMR of Z-3 (CDCl₃, 300 MHz) δ = 1.28 (t, 3H, J = 7.2 Hz), 1.59 (s, 3H), 1.97 (s, 3H), 4.19 (q, 2H, J = 7.2 Hz), 7.16 (m, 2H), 7.20 (m, 3H), 11.1 (bs, 1H) ppm. HRMS calculated for C₁₄H₁₈NO₃: 248.1281, found: 248.1279.

### Preparation Example 3

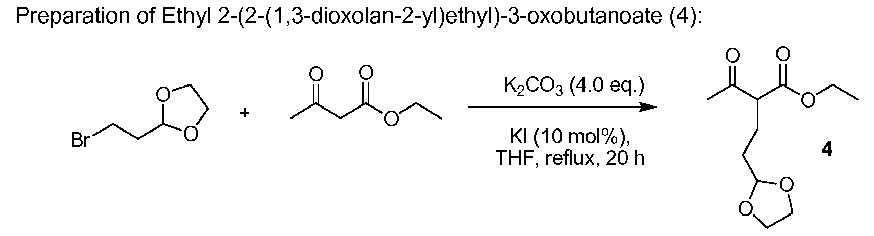

K₂CO₃ (4.1 g, 30 mmol, 4.0 eq.) was suspended in THF (10 mL) and ethyl acetoacetate (1.0 mL, 7.5 mmol) was added. Subsequently, 2-(2-bromoethyl)-1,3-dioxolane (0.97 mL, 8.3 mmol, 1.1 eq.) and KI (124 mg, 0.75 mmol, 10 mol%) were added. The resulting mixture was heated under refluxing conditions for 16 hours while stirring under a nitrogen atmosphere. The reaction mixture was cooled to room temperature and filtered to remove precipitated salts. The reaction mixture was quenched with HCl (1 M aq., 25 mL) and diluted with MTBE (25 mL). The aqueous layer was extracted with MTBE (25 mL) and the combined organic layers were washed with NaHCO₃ (sat. aq., 50 mL) and brine (sat. aq., 50 mL), dried (Na₂SO₄), filtered and concentrated in vacuo. The product was purified by column chromatography (n-heptane-EtOAc 4:1 v/v) to give 4 (190 mg, 0.83 mmol, 11 %) as an oil.

¹H-NMR of 4 (CDCl₃, 300 MHz) δ = 1.27 (t, 3H), 1.67 (m, 2H), 1.96 (m, 2H), 2.24 (s, 3H), 3.52 (m, 1H), 3.85 (m, 2H), 3.95 (m, 2H), 4.19 (m, 2H), 4.85 (m, 1H) ppm. HRMS calculated for C₁₁H₁₈O₅Na: 253.1046, found: 253.1056.

### Preparation Example 4

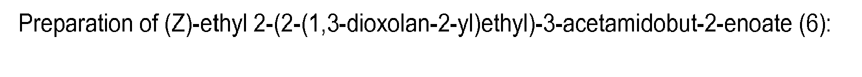

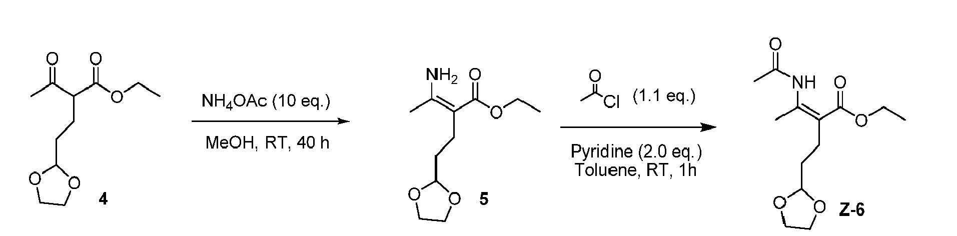

β-ketoester 4 (1.0 g, 4.4 mmol) and ammonium acetate (2.7 g, 44 mmol, 10 eq.) were dissolved in MeOH (15 mL) and the resulting mixture was stirred for 40 hours under a nitrogen atmosphere (GC showed approximately 85% conversion). The reaction mixture was concentrated in vacuo and the product was diluted with water (25 mL) and EtOAc (50 mL). The aqueous layer was extracted with EtOAc (2x 25 mL) and the combined organic layers were dried (Na₂SO₄), filtered and concentrated in vacuo to give enamine 5. This residue was dissolved in toluene (20 mL) and pyridine (0.87 mL, 8.8 mmol, 2.0 eq.) and acetyl chloride (0.35 mL, 4.84 mmol, 1.1 eq.) were added. The resulting solution was stirred under a nitrogen atmosphere for 1 hour and then quenched by addition of HCl (2M aq., 10 mL). The aqueous layer was extracted with EtOAc (2 x 15 mL) and the combined organic layers were washed with NaHCO₃ (sat. aq., 25 mL) and water (25 mL), dried (Na₂SO₄), filtered and concentrated in vacuo. The product was purified by column chromatography (n-pentane-EtOAc 4:1 v/v) to give Z-6 (70 mg, 0.26 mmol, 5.9% over 2 steps) as a colorless oil.

¹H-NMR of Z-6 (CDCl₃, 300 MHz) δ = 1.23 (t, 3H, J = 7.2 Hz), 1.67 (m, 2H) 2.04 (s, 3H), 2.34 (m, 2H), 2.38 (s, 3H), 3.79 (m, 2H), 3.89 (m, 2H), 4.13 (q, 2H, J = 7.2 Hz), 4.79 (m, 1H) ppm. HRMS calculated for C₁₃H₂₁NO₅Na: 294.1311, found: 294.1317.

### Preparation Example 5

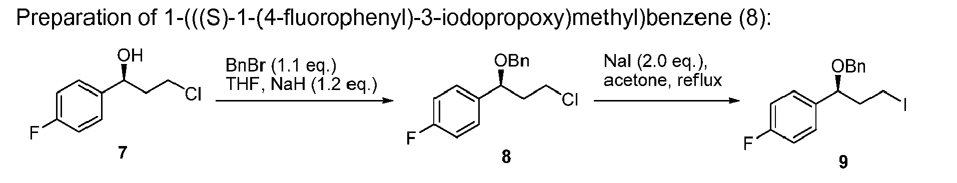

Compound 7 was prepared as described by: Xu, X.; Fu, R.; Chen, J.; Chen, S.; Bai, X. Bioorg. Med. Chem. Lett. (2007) 17, 101-104.

NaH (60% w/w in mineral oil, 1.27 g, 31.8 mmol, 1.2 eq.) was suspended in THF (50 mL) and a solution of chiral alcohol 7 (5.0 g, 26.5 mmol) in THF (50 mL) was added dropwise. Subsequently, benzylbromide (3.46 mL, 29.1 mmol, 1.1 eq.) was added and the resulting mixture was stirred at room temperature under a nitrogen atmosphere for 8 hours (GC showed 97% conversion). The reaction mixture was then poured into a mixture of ice and water (100 mL) and the layers were separated. The aqueous layer was extracted with EtOAc (2x 100 mL) and the combined organic layers were dried (Na₂SO₄) filtered and concentrated in vacuo. The product was purified by column chromatography (n-heptane-EtOAc 9:1 v/v) to give 8 (7.16 g, 25.7 mmol, 97%) as a colorless oil.

¹H-NMR of 8 (CDCl₃, 300 MHz) δ = 1.93 (m, 1H), 2.20 (m, 1H), 3.45 (dt, 1H, J = 5.7, 11.1 Hz), 3.66 (m, 1H), 4.20 (d, 1H, J = 12.0 Hz), 4.36 (d, 1H, J = 11.4 Hz), 4.50 (dd, 1H, J = 4.5, 9.0 Hz), 6.99 (t, 2H, J = 8.7 Hz), 7.24 (m, 7H) ppm.

Compound 8 (1.0 g, 3.6 mmol) was dissolved in acetone (7.0 mL) and Nal (1.1 gr, 7.2 mmol, 2.0 eq.) was added. The resulting solution was heated under reflux conditions for 71 hours, after which GC showed 98% conversion. The reaction mixture was cooled to room temperature and concentrated in vacuo. The product was dissolved in EtOAc (25 mL) and washed with water (25 mL). The organic layer was dried (Na₂SO₄), filtered and concentrated in vacuo to give 9 (1.3 g, 3.5 mmol, 98%) as a colorless oil.

¹H-NMR of 9 (CDCl₃, 300 MHz) δ = 2.00 (m, 1H), 2.22 (m, 1H), 3.10 (m, 1H), 3.23 (m, 1H), 4.20 (d, 1H, J = 11.4 Hz), 4.36 (d, 1H, J = 11.4 Hz), 4.38 (dd, 1H, J = 4.8, 8.1 Hz), 6.99 (t, 2H, J = 8.7 Hz), 7.23 (m, 7H) ppm.).

### Preparation Example 6

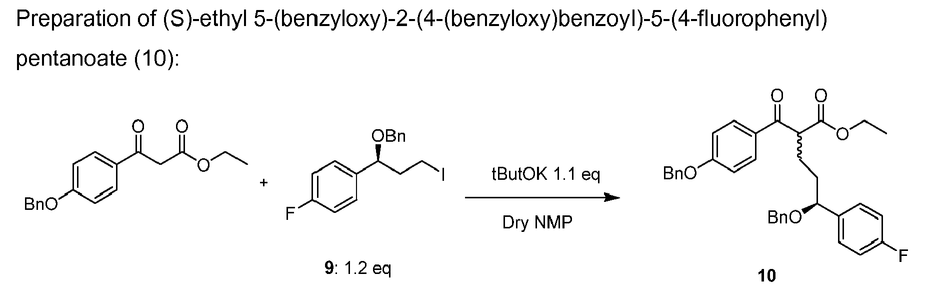

Compound 9 (2.98 g, 8.04 mmol, 1.2 eq.) was dissolved in N-methylpyrrolidinone (NMP, 10 mL) in a 3-necked flask under a nitrogen atmosphere. In another 3-necked flask, ethyl p-benzyloxybenzoylacetate (2.0 g, 6.7 mmol, 1.0 eq.) was dissolved in NMP (50 mL) under a nitrogen atmosphere, after which tBuOK (0.83 g, 7.4 mmol, 1.1 eq.) was added in small portions maintaining the temperature at 20 °C using a water bath. After completion of the addition of tBuOK, the reaction mixture was stirred for 10 minutes and subsequently it was transferred to the solution containing compound 9 by a cannula in a dropwise fashion over 2 hours. The resulting reaction mixture was stirred for 18 hours at ambient temperature under a nitrogen atmosphere. Subsequently, the reaction mixture was diluted with MTBE (100 mL) and water (50 mL). The layers were separated and the aqueous layer was extracted with MTBE (2 x 50 mL). The combined organic layers were washed with water (3 x 50 mL), dried (Na₂SO₄), filtered and concentrated in vacuo. The product was purified by column chromatography (n-heptane-EtOAc 98:2 v/v) to give 10 (2.4 g, 4.4 mmol, 65%) as an off-white solid.

¹H-NMR of 10 (CDCl₃, 300 MHz) δ = 1.07 (m, 3H), 1.54-2.16 (m, 4H), 4.03 (m, 2H), 4.11-4.36 (m, 4H), 5.05 (d, 2H, J = 4.5 Hz), 6.93 (m, 4H), 7.21 (m, 12H), 7.82 (dd, 2H, J = 2.1, 9.3 Hz) ppm.

### Preparation Example 7

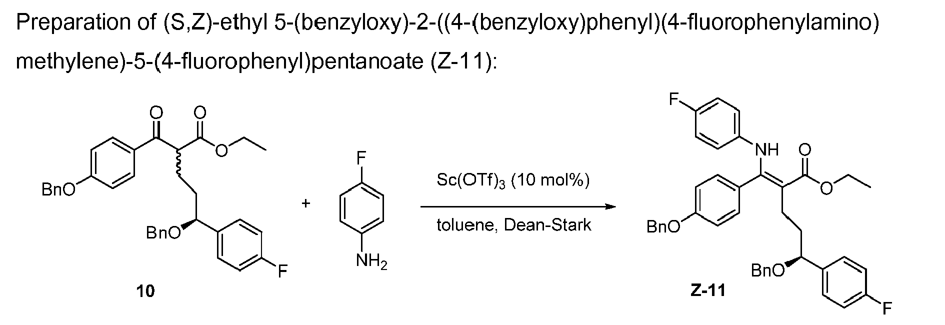

Ketone 10 (6.3 g, 11.7 mmol, 1.0 eq.) was dissolved in toluene (100 mL) and p-fluoro-aniline (2.24 mL, 23.3 mmol, 2.0 eq.) and Sc(OTf)₃ (0.58 g, 1.18 mmol, 10 mol%) were added. The resulting mixture was heated under reflux using Dean-Stark conditions for 16 hours and then cooled to room temperature. The solution was diluted with EtOAc (50 mL) and washed with water (2 x 100 mL). The organic layer was dried (Na₂SO₄), filtered and concentrated in vacuo. The product was purified by column chromatography (toluene) to give Z-11 (6.7 g, 10.7 mmol, 91 %) as an off-white solid.

¹H-NMR of Z-11 (CDCl₃, 300 MHz) δ = 1.31 (t, 3H, J = 7.2 Hz), 1.78 (m, 1H), 1.96 (m, 2H), 2.19 (m, 1H), 4.10-4.35 (m, 5H), 5.02 (s, 2H), 6.51 (dd, 2H, J = 5.1, 9.0 Hz), 6.71 (t, 2H, J = 8.7 Hz), 6.83 (d, 2H, J = 8.7 Hz), 7.00 (m, 4H), 7.14-7.50 (m, 13H) ppm.

### Preparation Example 8

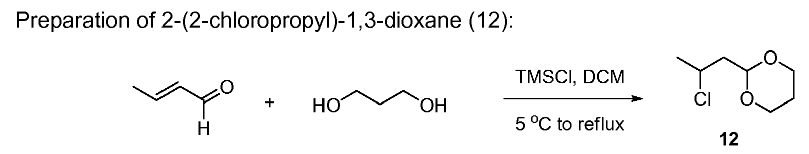

Trimethylsilylchloride (26.6 mL, 210 mmol, 2.1 eq.) was added dropwise over 45 minutes to a solution of 1,3-propanediol (14.4 mL, 200 mmol, 2.0 eq.) and crotonaldehyde (8.2 mL, 100 mmol, 1.0 eq.) in dichloromethane (50 mL) at 5 °C under a nitrogen atmosphere. After completion of the addition, the reaction mixture was allowed to reach room temperature and stirred for 15 minutes. Subsequently, the mixture was heated under reflux conditions for 2 hours and was then cooled to room temperature. The reaction mixture was diluted with Et₂O (100 mL) and NaHCO₃ (sat. aq.; 100 mL) and the layers were separated. The organic layer was washed with NaHCO₃ (sat. aq.; 50 mL) and brine (50 mL), dried (Na₂SO₄), filtered and concentrated in vacuo (T = 32 °C, P = 100 mbar). Concentration was stopped when 25 grams of product remained. This crude product was purified by distillation under reduced pressure (T = 60 °C, P = 5 mbar) to give 12 (12.8 g, 77.8 mmol, 78%) as a colorless liquid.

¹H-NMR of 12 (CDCl₃, 300 MHz) δ = 1.34 (d, 1H, J = 13.4 Hz), 1.51 (d, 3H, J = 6.7 Hz), 1.93-2.08 (m, 3H), 3.78 (t, 2H, J = 12.0 Hz), 4.07-4.20 (m, 3H), 4.72 (t, 1H, J = 6.3 Hz) ppm.

¹³C-NMR of 12 (CDCl₃, 75 MHz): 23.7, 23.9, 43.6, 51.9, 64.9, 65.0, 98.0 ppm.

### Preparation Example 9

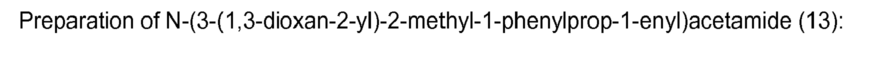

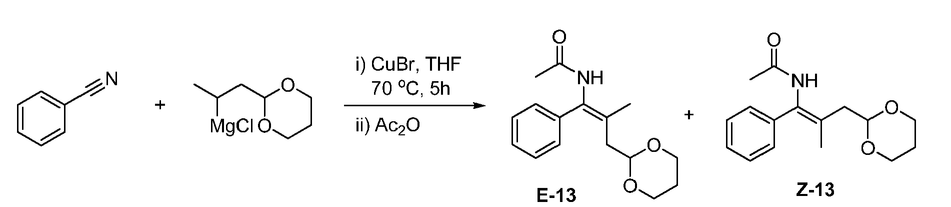

Preparation of the Grignard reagent of 12: Mg (2.37 g, 97.3 mmol, 1.3 eq.) was charged in a 3-necked flask equipped with a dropping funnel and a condenser. The experimental set-up was dried by heating under vacuum followed by cooling under a nitrogen atmosphere (3 cycles). Subsequently, a solution of 12 (1.2 g, 7.5 mmol, 0.1 eq.) in THF (9.0 mL) was added and the resulting mixture was heated with a hotgun while stirring vigorously under a nitrogen atmosphere. When an exothermic reaction was observed (start of the formation of the Grignard reagent), a solution of 12 (11.1 g, 67.5 mmol, 0.9 eq.) in THF (81 mL) was added at such a rate that reflux conditions were maintained without additional heating (approximately 45 minutes). Upon completion of the addition, the reaction mixture was heated at 60 °C for 1 hour. Subsequently, the stirring was stopped and the Grignard solution was left to settle for 16 hours.

Titration: the concentration of the Grignard solution was determined to be 0.82 M by titration with sec-butanol in the presence of catalytic 1,10-phenanthroline.

Addition of the Grignard reagent to benzonitrile: The Grignard solution of 12 (25 mL, 0.82 M, 20 mmol, 1.0 eq.) was added to CuBr (0.13 g, 0.91 mmol, 4.5 mol%) after which benzonitrile (3.9 mL, 38 mmol, 1.9 eq.) was added. The resulting black suspension was stirred at 70 °C under a nitrogen atmosphere for 5 hours and then quenched by the addition of acetic anhydride (3.7 mL, 40 mmol, 2.0 eq.). The reaction mixture was stirred for another 45 minutes at 70 °C and then cooled to room temperature. The solution was diluted with EtOAc (50 mL) and ammonia (aq. 10 wt%, 50 mL) resulting in a brown/green suspension. Toluene (50 mL) and brine (50 mL) were added resulting in two clear phases. The phases were separated and the organic layer was washed with NH₃ (aq. 10 wt%, 25 mL), NH₄Cl (sat. aq., 25 mL) and brine (25 mL), dried (Na₂SO₄), filtered and concentrated in vacuo to give a brown oil (crude ¹H-NMR shows a Z-13/E-13 ratio of 2:1 The product was purified by column chromatography (n-heptane-EtOAc 3:7 to 1:9 v/v) to give a mixture of E-13 and Z-13 (3.5 g, 13 mmol, 63%) as an off-white solid. Z-13 was further purified by crystallization from a toluene/n-heptane mixture. The mother liquor was concentrated in vacuo and the remaining solid was purified by column chromatography (toluene-acetone 3:1 to 2:1 v/v) to obtain E-13.

¹H-NMR of Z-13 (CD₃OD, 300 MHz) δ = 1.26 (d, 1H, J = 13.4 Hz), 1.67 (s, 3H), 1.88 (s, 3H), 1.92 (m, 1H), 2.33 (d, 2H, J = 5.3 Hz), 3.71 (dt, 2H, J = 2.1, 12.0 Hz), 4.0 (ddd, 2H, J = 1.2, 5.0, 11.9 Hz), 4.67 (t, 1H, J = 5.3 Hz), 7.14 (m, 5H) ppm. ¹³C-NMR of Z-13 (CD₃OD, 75 MHz): 20.4, 23.2, 27.3, 41.1, 68.4, 68.5, 103.3, 128.7, 129.3, 129.3, 129.8, 130.6, 130.6, 133.0, 140.2, 172.3 ppm.

¹H-NMR of E-13 (CD₃OD, 300 MHz) δ = 1.20 (d, 1H, J = 13.4 Hz), 1.70 (s, 3H), 1.85 (m, 1H), 1.87 (s, 3H), 2.27 (d, 2H, J = 5.7 Hz), 3.71 (dt, 2H, J = 2.5, 12.4 Hz), 4.0 (ddd, 2H, J = 1.2, 5.0, 11.7 Hz), 4.62 (t, 1H, J = 5.3 Hz), 7.14 (m, 5H) ppm.

¹³C-NMR of E-13 (CD₃OD, 75 MHz): 19.1, 23.1, 27.3, 41.1, 68.4, 68.5, 103.1, 128.7, 129.3, 129.3, 129.8, 130.6, 130.6, 132.9, 140.3, 172.0 ppm.

Discrimination between the E- and Z-isomer was univocally established by 2D-NMR. In particular, the Z-isomer was identified by a NOE-interaction between an ortho-hydrogen on the aromatic ring and a hydrogen of the methyl-group on the olefin as depicted in the figure below:

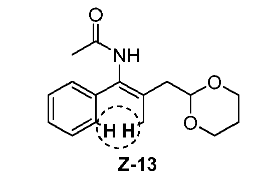

### Preparation Example 10

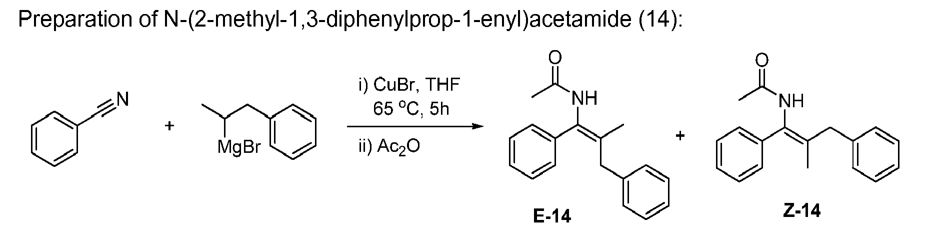

Preparation of the Grignard reagent: Mg (4.0 g, 163 mmol, 1.3 eq.) was charged in a 3-necked flask equipped with a dropping funnel and a condenser. The experimental set-up was dried by heating under vacuum followed by cooling under a nitrogen atmosphere (3 cycles). Subsequently, a solution of 2-bromo-1-phenylpropane (2.0 mL, 1.3 mmol, 0.1 eq.) in THF (25 mL) was added and the resulting mixture was heated with a hotgun while stirring vigorously under a nitrogen atmosphere. When an exothermic reaction was observed (start of the formation of the Grignard reagent), a solution of 2-bromo-1-phenylpropane (18 g, 117 mmol, 0.9 eq.) in THF (225 mL) was added at such a rate that reflux conditions were maintained without additional heating (approximately 1 hour). Upon completion of the addition, the reaction mixture was heated at 60 °C for 1 hour. Subsequently, the stirring was stopped and the Grignard solution was left to settle for 16 hours.

Titration: the concentration of the Grignard solution was determined to be 0.40 M by titration with sec-butanol in the presence of catalytic 1,10-phenanthroline.

Addition of the Grignard reagent to benzonitrile: The Grignard solution of 2-bromo-1-phenylpropane (120 mL, 0.40 M, 48 mmol, 1.1 eq.) was added to CuBr (313 mg, 2.18 mmol, 5.0 mol%) after which benzonitrile (4.5 mL, 44 mmol, 1.0 eq.) was added. The resulting black suspension was stirred at 65 °C under a nitrogen atmosphere for 16 hours and then quenched by the addition of acetic anhydride (4.5 mL, 48 mmol, 1.1 eq.). The reaction mixture was stirred for another hour at 65 °C and then cooled to room temperature. The solution was diluted with water (200 mL) and EtOAc (200 mL) resulting in a biphasic system with two clear phases. The phases were separated and the organic layer was washed with NH₃ (10 wt% aq., 200 mL), NH₄Cl (sat. aq., 200 mL) and brine (200 mL), dried (MgSO₄), filtered and concentrated in vacuo to give a brown oil (crude ¹H-NMR shows a Z-14/E-14 ratio of 1:3). The product was purified by column chromatography (n-pentane-EtOAc 1:1 to 2:3 v/v) to give a mixture of E-14 and Z-14 (7.9 g, 30 mmol, 68%) as slightly yellow solid. E-14 was further purified by crystallization from toluene. An analytical sample of pure Z-14 was obtained via column chromatography (n-pentane-EtOAc 2:3 v/v).

¹H-NMR of E-14 (CDCl₃, 300 MHz) δ = 1.63, 1.71 (2 x s, 3H (rotamers), 1.97 (s, 3H), 3.42, 3.47 (2 x s, 2H, rotamers), 6.68 (bs, 1H), 7.10-7.28 (m, 10H) ppm.

¹³C-NMR of E-14 (CDCl₃, 75 MHz): 18.6, 23.8, 40.4, 126.4, 128.0, 128.7, 128.7, 128.8, 128.8, 128.9, 128.9, 129.0, 129.0, 132.0, 140.1, 168.3 ppm.

¹H-NMR of Z-14 (CDCl₃, 300 MHz) δ = 1.62, 1.74 (2 x s, 3H (rotamers), 1.93 (s, 3H), 3.46, 3.53 (2 x s, 2H, rotamers), 6.63 (bs, 1H), 7.14-7.28 (m, 10H) ppm.

¹³C-NMR of Z-14 (CDCl₃, 75 MHz): 19.1, 23.2, 39.9, 126.0, 128.0, 128.7, 128.7, 128.8, 128.8, 128.9, 128.9, 129.0, 129.0, 132.2, 139.4, 168.6 ppm.

Discrimination between the E- and Z-isomer was univocally established by 2D-NMR. In particular, the isomers were identified by NOE-interactions between the NH-proton and the hydrogens on the benzylic (Z) or methyl (E) position as depicted in the figure below:

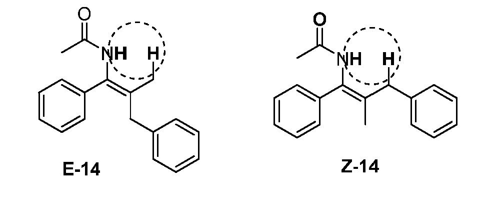

### Preparation Example 11

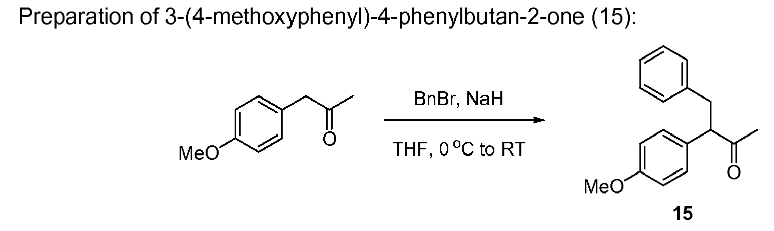

NaH (60 wt% in oil, 2.44 g, 60.8 mmol, 1.0 eq.) was added to a solution of p-methoxyphenylacetone (10.0 g, 60.9 mmol, 1.0 eq.) in THF (100 mL) at 0 °C under a nitrogen atmosphere. Subsequently, benzylbromide (7.24 mL, 60.8 mmol, 1.0 eq.) was added at 0 °C and the resulting mixture was stirred for 72 hours under a nitrogen atmosphere at room temperature. The reaction mixture was quenched by the addition of HCl (1 M aq., 50 mL) and diluted with EtOAc (100 mL). The layers were separated and the aqueous layer was extracted with EtOAc (50 mL). The combined organic layers were washed with NaHCO₃ (sat. aq., 100 mL) and brine (100 mL), dried (MgSO₄), filtered and concentrated in vacuo. The product was purified by column chromatography (n-heptane-EtOAc 9:1 v/v) to give 15 (12.4 g, 48.8 mmol, 80.2%) as an off-white solid.

¹H-NMR of 15 (CDCl₃, 300 MHz) δ = 2.03 (s, 3H), 2.88 (dd, 1H, J = 7.5, 13.8 Hz), 3.40 (dd, 1H, J = 7.5, 13.8 Hz), 3.80 (s, 3H), 3.87 (t, 1H, J = 7.4 Hz), 6.85 (d, 2H, J = 8.8 Hz), 7.07 (m, 4H), 7.19 (m, 3H) ppm.

### Preparation Example 12

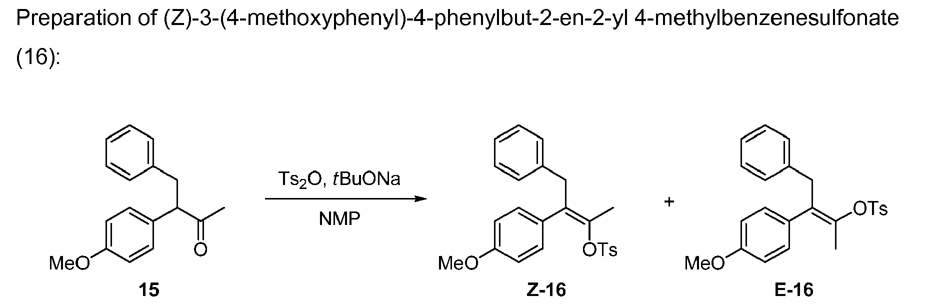

The method followed was analogous to Wallace, D. J.; Klauber, D. J.; Chen, C. -Y.; Volante, R. P. Org. Lett. (2003) 5, 4749-4752.

Ketone 15 (6.0 g, 24 mmol) was dissolved in N-methyl-2-pyrrolidinone (NMP, 50 mL) under a nitrogen atmosphere at room temperature. Subsequently, the solution was cooled to 0 °C, after which sodium tertbutoxide (2.72 g, 28.3 mmol, 1.2 eq.) was added in a single portion. The reaction mixture was then stirred for 90 minutes while being allowed to warm to room temperature. Subsequently, the solution was cooled to -20 °C and p-toluenesulfonic anhydride (9.25 g, 28.3 mmol, 1.2 eq.) was added in a single portion. The reaction was stirred for 1.5 hours while maintaining the temperature between - 20 °C and -10 °C and then quenched with NaHCO₃ (sat. aq., 50 mL) and diluted with MTBE (100 mL). The resulting mixture was diluted with water (100 mL) until a clear biphasic system was obtained. The layers were then separated and the aqueous layer was extracted with MTBE (100 mL). The combined organic layers were washed with water (100 mL) and brine (100 mL), dried (Na₂SO₄), filtered and concentrated in vacuo. ¹H-NMR of the crude product showed that the ratio of starting material to product was 23: 77 (15 : Z-16 : E-16 = 20 : 40 : 40). The product was purified by column chromatography (n-pentane-EtOAc 9:1 to 4:1 v/v) to give 16 (1:1 E/Z mixture, 7.5 g, 18 mmol, 77%) as a yellow oil.

¹H-NMR of Z-16 (CDCl₃, 300 MHz) δ = 2.22 (s, 3H), 2.28 (s, 3H), 3.57 (s, 2H), 3.76 (s, 3H), 6.50 (d, 2H, J = 9.0 Hz), 6.75 (d, 2H, J = 8.7 Hz), 6.96 (t, 4H, J = 8.7 Hz), 7.13 (m, 3H), 7.24 (d, 2H, J = 8.4 Hz) ppm.

¹H-NMR of E-16 (CDCl₃, 300 MHz) δ = 1.95 (s, 3H), 2.47 (s, 3H), 3.58 (s, 2H), 3.76 (s, 3H), 6.76 (d, 2H, J = 8.7 Hz), 6.87 (d, 2H, J = 9.0 Hz), 6.98 (d, 2H, J = 8.1 Hz), 7.14 (m, 3H), 6.36 (d, 2H, J = 8.4 Hz), 7.89 (d, 2H, J = 8.4 Hz) ppm.

Discrimination between the E- and Z-isomer was univocally established by 2D-NMR. In particular, the E-isomer was identified by the NOE-interactions between the protons of the p-MeO-phenyl-substituent and the hydrogens of the vinylic methyl-substituent as depicted in the figure below:

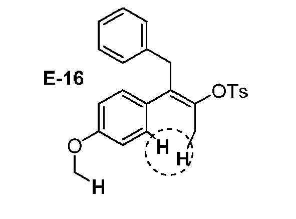

### Preparation Example 13

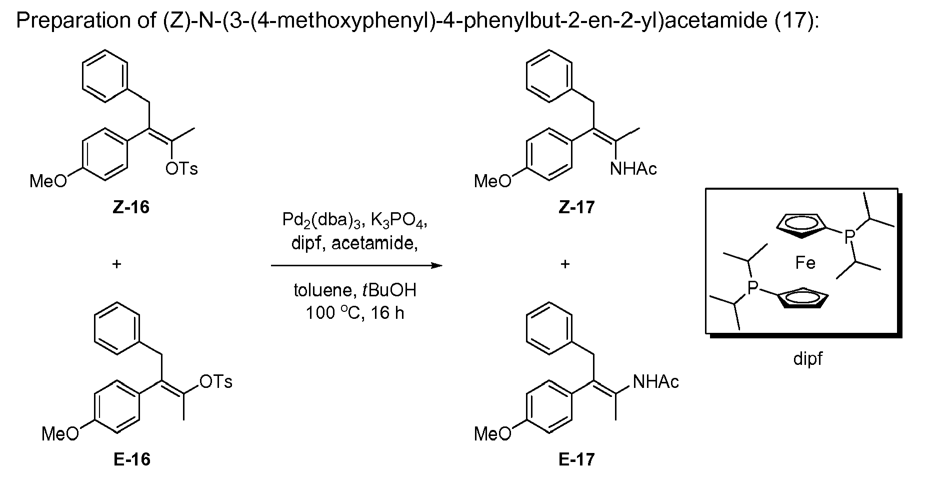

The method followed was analogous to Wallace, D. J.; Klauber, D. J.; Chen, C. -Y.; Volante, R. P. Org. Lett. (2003) 5, 4749-4752.

1,1'-Bis(diisopropylphosphino)ferrocene (dipf, 0.53 g, 1.3 mmol, 8.0 mol%), K₃PO₄ (6.8 g, 32 mmol, 2.0 eq.) and Pd₂(dibenzylideneacetone)₃ (0.29 g, 0.32 mmol, 2.0 mol%) were charged in a dry Schlenk tube under a nitrogen atmosphere. In a second dry Schlenk tube, 16 (E/Z = 1:1, 6.6 g, 16 mmol, 1.0 eq.) was dissolved in a mixture of dry toluene (24 mL) and dry tAmOH (16 mL). The resulting solution was degassed and stirred in the presence of molsieves (4A) for 15 minutes under a nitrogen atmosphere. The solution was then transferred to the first Schlenk tube upon which a dark purple mixture was formed. This mixture was stirred for 5 minutes under a nitrogen atmosphere at room temperature, after which acetamide (1.9 g, 32 mmol, 2.0 eq.) was added. The resulting green suspension was stirred at 100 °C in a sealed system for 16 hours. The reaction mixture was cooled to room temperature, filtered over silica (eluent: EtOAc) and concentrated in vacuo. ¹H-NMR (CDCl₃) showed complete conversion of Z-16 and 50% conversion of E-16. The product was purified by column chromatography (n-heptane-EtOAc 1:1 to 2:3 to 1:2 v/v) to give 17 (E/Z-mixture of 1:2, 3.3 g, 11 mmol, 68%) as an off-white solid. The minor isomer E-17 was purified by crystallization from toluene, while an analytical sample of the major isomer Z-17 was obtained by column chromatography (n-heptane-EtOAc 1:1 to 2:3 v/v).

¹H-NMR of Z-17 (CD₃OD, 300 MHz) δ = 1.80 (s, 3H), 2.13 (s, 3H), 3.74 (s, 3H), 3.78 (s, 2H), 6.78 (d, 2H, J = 8.8 Hz), 7.20 (d, 2H, J = 8.8 Hz), 7.15 (m, 5H) ppm.

¹³C-NMR of Z-17 (CD₃OD, 75 MHz): 18.6, 22.9, 40.7, 56.0, 114.8, 114.8, 114.8, 127.4, 129.7, 129.7, 130.0, 130.0, 131.1, 131.1, 134.6, 134.9, 140.9, 160.4, 172.7 ppm.

¹H-NMR of E-17 (CD₃OD, 300 MHz) δ = 1.80 (s, 3H), 2.05 (s, 3H), 3.68 (s, 2H), 3.74 (s, 3H), 6.79 (d, 2H, J = 8.8 Hz), 6.97 (d, 2H, J = 8.8 Hz), 7.09 (m, 5H) ppm.

¹³C-NMR of E-17 (CD₃OD, 75 MHz): 19.7, 23.2, 40.9, 56.0, 114.8, 114.8, 114.8, 127.2, 129.5, 129.5, 130.4, 130.4, 131.6, 131.6, 134.6, 136.9, 141.0, 160.3, 172.8ppm.

### Example 1

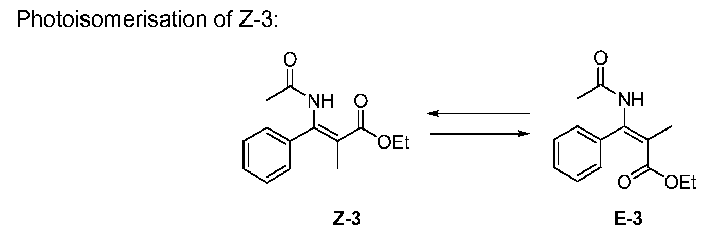

Enamide Z-3 (200 mg, 0.808 mmol) was dissolved in toluene (320 mL) and the resulting solution was stirred at 20 °C under a nitrogen atmosphere while irradiating with a medium pressure Hg-lamp. The isomerisation was monitored using HPLC.

The equilibrium was reached after approximately 30 minutes. The ratio between E-3 and Z-3 was 80:20 at this point. After 5 hours, the reaction mixture was concentrated in vacuo. The mixture of enamides was purified by column chromatography (n-heptane-EtOAc 3:1) to give E-3 (120 mg, 0.485 mmol, 60%) as an off-white solid.

¹H-NMR of E-3 (CDCl₃, 300 MHz) δ = 0.76 (t, 3H, J = 7.2 Hz), 1.95 (bs, 6H), 3.82 (q, 2H, J = 7.2 Hz), 6.64 (bs, 1H), 7.23 (m, 5H) ppm.

HPLC method to determine the E/Z-ratio: Column Nucleosil 120-3-C₁₈, 250 x 4.6 mm ID, particle size 3 µm, eluent: H₂O-CH₃CN, gradient: 60:40 (v/v) to 5:95 over 15 min, then to 60:40 over 0.1 min (t = 15.1 min), maintain at 60:40 for 4.9 min (t = 20 min). Flow: 1.0 mL/min, temperature: 40 °C, UV detection at 220 nm. Retention time E-3 = 8.7 min. Retention time Z-3 = 12.3 min.

### Example 2

Example 1 was repeated but using solvents other than THF. The results are given below.

| Solvent | Time to equilibrium (min.) | E/Z ratio at equilibrium |
|---|---|---|
| Toluene | 30 | 80/20 |
| 1,4-Dioxane | 50 | 70/30 |
| Acetone | 30 | 69/31 |
| EtOAc | 30 | 75/25 |
| dimethylcarbonate | 25 | 72/28 |
| THF | 30 | 63/37 |
| MeOH | 15 | 80/20 |
| Dichloromethane | degradation | 60/40 |

### Example 3

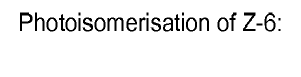

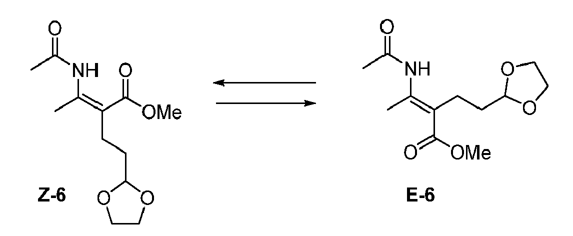

Enamide Z-6 (70 mg, 0.26 mmol) was dissolved in MeOH (320 mL) and the resulting solution was stirred at 20 °C under a nitrogen atmosphere while irradiating with a medium pressure Hg-lamp (Reactor B). The isomerisation was monitored using HPLC, showing an E/Z-ratio of 55/45 after 90 minutes.

HPLC method to determine the E/Z-ratio: Column Nucleosil 120-3-C₁₈, 250 x 4.6 mm ID, particle size 3 µm, eluent: H₂O-CH₃CN, gradient: 60:40 (v/v) to 5:95 over 15 min, then to 60:40 over 0.1 min (t = 15.1 min), maintain at 60:40 for 4.9 min (t = 20 min). Flow: 1.0 mL/min, temperature: 40 °C, UV detection at 220 nm. Retention time Z-6 = 9.8 min. Retention time E-6 = 14.1 min.

### Example 4

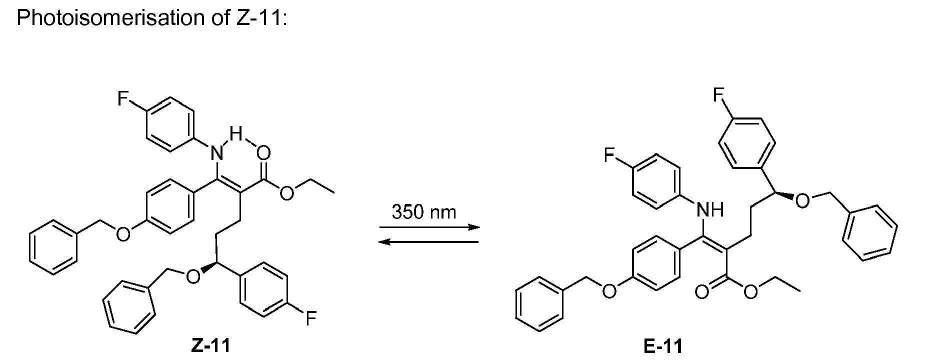

A solution of enamide Z-11 (7.5 mg, 0.012 mmol) in iPrOH (25 mL) was irradiated with 350 nm UV light (Reactor A) at room temperature for 2 hours. Analysis by HPLC showed that the photoequilibrium was reached after 30 minutes with an E/Z-ratio of 60/40 with < 5% degradation.

HPLC method to determine the E/Z-ratio: Column Nucleosil 120-3-C₁₈, 250 x 4.6 mm ID, particle size 3 µm, eluent: H₂O-CH₃CN, gradient: 60:40 (v/v) to 5:95 over 15 min, maintain at 5:95 for 5 min (t =20 min), then to 60:40 over 5 min (t = 25 min), maintain at 60:40 for 5 min (t = 30 min). Flow: 1.0 mL/min, temperature: 40 °C, UV detection at 220 nm. Retention time E-11 = 19.0 min. Retention time Z-11 = 21.2 min.

### Example 5

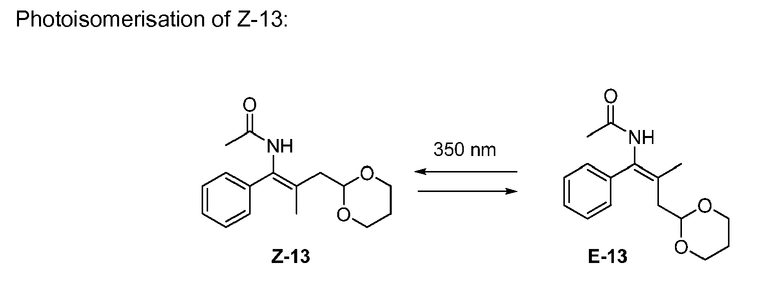

A solution of enamide Z-13 (7.5 mg, 0.027 mmol) in MeOH (25 mL) was irradiated with 350 nm UV light (Reactor A) in the presence of propiophenone as a sensitizer (3.6 mg, 0.027 mmol, 1.0 eq.) at ambient temperature. Analysis by HPLC showed that the photoequilibrium was reached after 10 minutes with an E/Z-ratio of 45/55. HPLC method to determine the E/Z-ratio: Column Nucleosil 120-3-C₁₈, 250 x 4.6 mm ID, particle size 3 µm, eluent: H₂O-CH₃CN, gradient: 60:40 (v/v) to 5:95 over 15 min, then to 60:40 over 0.1 min (t = 15.1 min), maintain at 60:40 for 4.9 min (t = 20 min). Flow: 1.0 mL/min, temperature: 40 °C, UV detection at 220 nm. Retention time E-13 = 9.52 min. Retention time Z-13 = 10.80 min.

### Example 6

Example 5 was repeated using different sensitizers (1 eq. of sensitizer unless stated otherwise):

| Sensitizer | Result (E/Z equilibrium position; degradation) |
|---|---|
| None (toluene solvent) | No conversion |
| Phenol | No conversion |
| Acetone | No conversion |
| Acetone (solvent) | E/Z = 32/68, 40% degradation* |
| Benzonitrile | No conversion |
| Aniline | No conversion |
| Propiophenone | E/Z = 45/55, 1% degradation* |
| Xanthone | Degradation |
| Anthrone | Degradation |
| Carbazole | E/Z = 43/57, 6% degradation* |
| Benzophenone | E/Z = 48/52, 13% degradation* |
| Anthraquinone | E/Z = 22/78, 70% degradation* |
| Naphthalene | No Conversion |
| Benzil | No Conversion |
| Acetophenone | E/Z 44/56, 1% degradation* |
| 4-Chloroacetophenone | E/Z 46/54, slow degradation* |
| 4-Methoxyacetophenone | No Conversion |
| 4-trifluoromethylacetophenone | No Conversion |
| 2,4-Difluoropropiophenone | E/Z 48/52, 2% degradation* |
| Butyrophenone | E/Z = 45/55, 1% degradation* |
| Benzylideneacetophenone | No Conversion |

| | |
|---|---|
| * degradation in mol% as compared to the amount of starting Z-13. | |

### Example 7

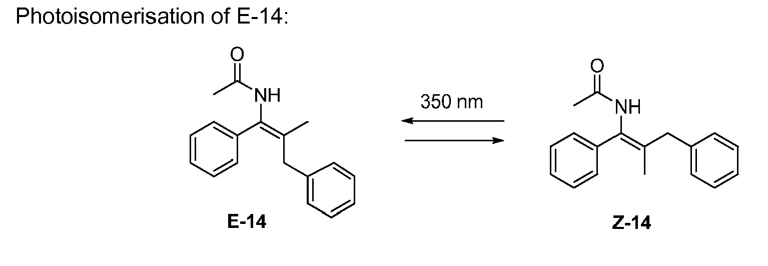

A solution of enamide E-14 (7.5 mg, 0.028 mmol) in MeOH (25 mL) was irradiated with 350 nm UV light (Reactor A) in the presence of propiophenone as a sensitizer (3.8 mg, 0.028 mmol, 1.0 eq.) at ambient temperature. Analysis by HPLC showed that the photoequilibrium was reached after 60 minutes with an E/Z-ratio of 43/57 and < 5% degradation.

HPLC method to determine the E/Z-ratio: Column Nucleosil 120-3-C₁₈, 250 x 4.6 mm, particle size 3 µm, eluent: H₂O-CH₃CN, gradient: 60:40 (v/v) to 5:95 over 15 min, then to 60:40 over 0.1 min (t = 15.1 min), maintain at 60:40 for 4.9 min (t = 20 min). Flow: 1.0 mL/min, temperature: 40 °C, UV detection at 220 nm. Retention time E-14 = 12.3 min. Retention time Z-14 = 12.5 min.

### Example 8

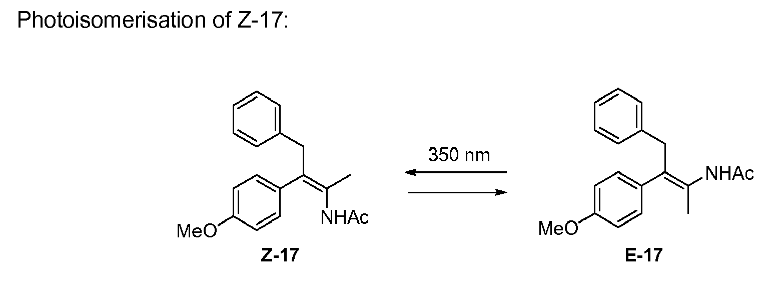

A solution of enamide Z-17 (7.5 mg, 0.025 mmol) in MeOH (25 mL) was irradiated with 350 nm UV light (Reactor A) in the presence of propiophenone as a sensitizer (3.4 mg, 0.025 mmol, 1.0 eq.) at ambient temperature. Analysis by HPLC showed that the photoequilibrium was reached after 60 minutes with an E/Z-ratio of 80/20 and <5% of degradation.

HPLC method to determine the E/Z-ratio: Column Nucleosil 120-3-C₁₈, 250 x 4.6 mm ID 3 µm, eluent: H₂O-CH₃CN, gradient: 60:40 (v/v) to 5:95 over 15 min, then to 60:40 over 0.1 min (t = 15.1 min), maintain at 60:40 for 4.9 min (t = 20 min). Flow: 1.0 mL/min, temperature: 40 °C, UV detection at 220 nm. Retention time E-17 = 11.8 min. Retention time Z-17 = 12.1 min.

### Example 9

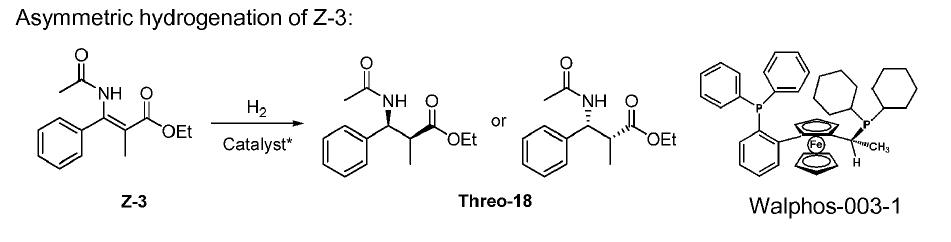

Rh(COD)₂BF₄ (4.0 mg, 10 µmol) and (S)-1-{(S_{P})-2-[2-(diphenylphosphino)phenyl]ferrocenyl}ethyldicyclohexylphosphine (Walphos-003-1; 7.6 mg, 11 µmol) were dissolved in DCM (0.5 mL) and stirred for 15 minutes. Subsequently, this solution was added to a solution of enamide Z-3 (49.5 mg, 0.200 mmol) in DCM (4.5 mL). The resulting mixture was stirred under a hydrogen atmosphere (25 bars) at 60 °C for 24 hours. HPLC-analysis of the crude reaction mixture showed that the desired threo-13 was formed in 90% yield (100% conversion) with an enantiomeric excess of 92%.

Ee determination was performed by GC using a Chirasil column DEX L-Val; T = 175 °C for 10 min, retention times: 5.98 min (Erithro-18-1), 6.24 min (Erithro-18-2) 7.50 min (Threo-18-1) and 7.88 min (Threo-18-2).

### Example 10

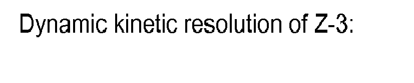

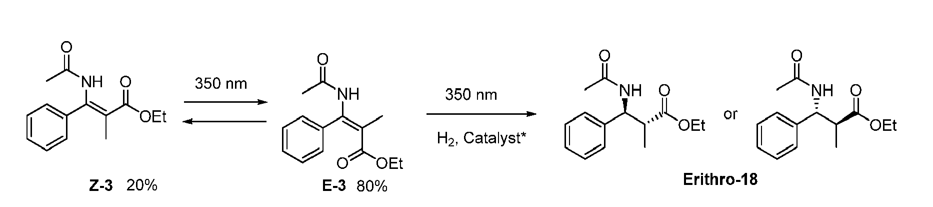

A stock solution of hydrogenation catalyst was prepared as follows: Rh(COD)₂BF₄ (6.2 mg, 15 µmol 1.0 eq.) and (S)-1-{(S_{P})-2-[2-(diphenylphosphino) phenyl]ferrocenyl}ethyldicyclohexylphosphine (Walphos-003-1; 11.3 mg, 17 µmol 1.1 eq.) were dissolved in DCM (2.5 mL) and stirred for 15 minutes under a nitrogen atmosphere.

A solution of Z-3 (22 mg, 89 µmol) in toluene (6.8 mL) was irradiated with UV light (350 nm) for 2 hours at room temperature. Subsequently, the catalyst stock solution (0.72 mL, 4.4 µmol, 5 mol%) was added to the reaction mixture and the resulting solution was stirred for 3 hours under a hydrogen atmosphere (1 bar) at room temperature while continuing to irradiate with UV-light (350 nm). After 3 hours the irradiation was stopped and the reaction mixture was concentrated in vacuo to give a yellow oil. Analysis of the crude mixture by ¹H-NMR showed > 90% conversion to 18. The diastereomeric excess was determined by ¹H-NMR to be >95% in favor of erithro-18. The enantiomeric excess was determined by GC to be 81%: Chirasil column DEX L-Val; T = 175 °C for 10 min, retention time 5.98 min (Erithro-18-1), 6.24 min (Erithro-18-2) 7.50 min (Threo-18-1) and 7.88 (Threo-18-2) min.

### Example 11

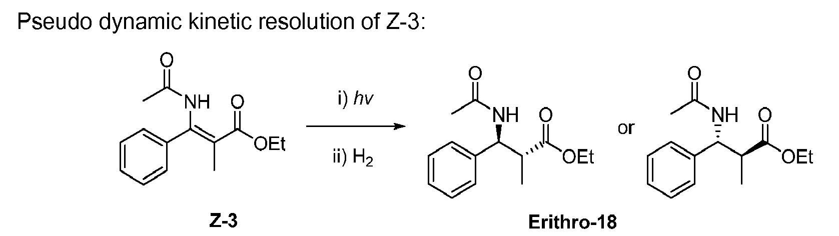

A stock solution of hydrogenation catalyst was prepared as follows: Rh(COD)₂BF₄ (65 mg, 0.16 mmol, 1.0 eq.) and (S)-1-{(S_{P})-2-[2-(diphenylphosphino) phenyl]ferrocenyl}ethyldicyclohexylphosphine (Walphos-003-1; 118 mg, 0.176 mmol, 1.1 eq.) were dissolved in DCM (8.0 mL) and stirred for 15 minutes under a nitrogen atmosphere.

A solution of Z-3 (1.0 g, 4.0 mmol) in toluene (320 mL) was irradiated with a medium pressure Hg-lamp for 3 hours at 20 °C under a nitrogen atmosphere. Subsequently, the irradiation was terminated and the catalyst stock solution (2.0 mL, 40 µmol, 1.0 mol%) was added to the reaction mixture. The resulting solution was stirred for 90 minutes under a hydrogen atmosphere (1 bar), after which the irradiation was repeated for 75 minutes. The irradiation was stopped and more catalyst stock solution was added (4.0 mL, 80 µmol, 2.0 mol%). After stirring for 60 minutes under a hydrogen atmosphere (1 bar), the solution was irradiated once more for 40 minutes. Subsequently, the irradiation was stopped and the remaining catalyst stock solution (2.0 mL, 40 µmol, 1.0 mol%) was added. The reaction mixture was stirred under a hydrogen atmosphere (1 bar) for 90 minutes and then concentrated in vacuo to give Erithro-18 (0.95 g, 3.8 mmol, 95% yield, 82% e.e.) as a yellow oil. The progress of the reaction (E/Z-ratio, yield of Erithro-18, diastereomeric and enantiomeric excess) was monitored using HPLC and GC. The E/Z-ratio and the chemical yield of Erithro-18 over time are schematically depicted below. E.e. and d.e. determination by GC: Chirasil column DEX L-Val; T = 175 °C for 10 min, retention time 5.98 min (Erithro-18-1), 6.24 min (Erithro-18-2), 7.50 min (Threo-18-1) and 7.88 (Threo-18-2) min.

Results are illustrated in Figure 3.

### Example 12

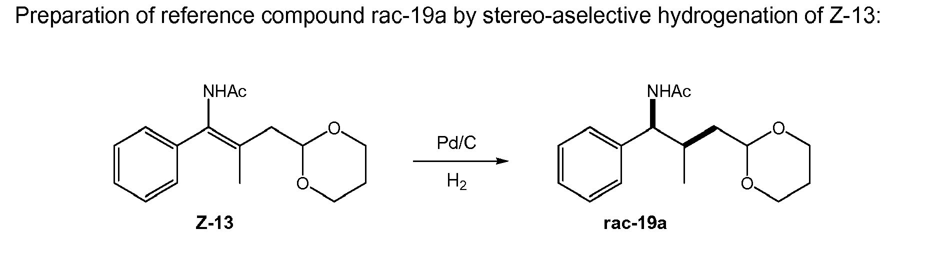

To enamide Z-13 (100 mg, 0.36 mmol) and Pd/C (10 wt%, 35 mg) was added EtOH (2.0 mL) and the resulting mixture was stirred under a hydrogen atmosphere (1 bar) at room temperature for 16 hours. Subsequently, the suspension was filtered over dicalite and concentrated in vacuo to give racemic 19a as an off-white solid (100 mg, 0.36 mmol, quantitative).

¹H-NMR of 19a (CDCl₃, 300 MHz): δ = 0.84 (d, 3H, J = 6.9 Hz), 1.26 (m, 2H), 1.57 (m, 2H), 1.91 (s, 3H), 1.94 (m, 1H), 2.14 (m, 1H), 3.65 (m, 2H), 3.99 (dd, 2H, J = 4.2, 11.4 Hz), 4.46 (t, 1H, J=5.1 Hz), 4.84 (m, 1H), 6.65 (bs, 1H), 7.13-7.21 (m, 5H) ppm.

¹³C-NMR of 19a (CDCl₃, 75 MHz): 17.0, 23.7, 26.1, 33.9, 39.0, 57.6, 67.2, 67.2, 101.3, 127.8, 127.8, 127.9, 128.7, 128.7, 141.1, 169.6 ppm.

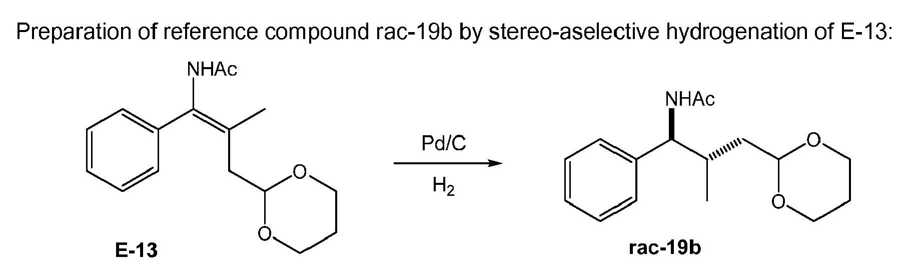

To enamide E-13 (100 mg, 0.36 mmol) and Pd/C (10 wt%, 35 mg) was added EtOH (2.0 mL) and the resulting mixture was stirred under a hydrogen atmosphere (1 bar) at room temperature for 16 hours. Subsequently, the suspension was filtered over dicalite and concentrated in vacuo to give racemic 19b as an off-white solid (100 mg, 0.36 mmol, quantitative).

¹H-NMR of 19b (CDCl₃, 300 MHz): δ = 0.75 (d, 3H, J = 6.9 Hz), 1.28 (m, 2H), 1.42 (m, 1H), 1.81 (m, 1H), 1.91 (s, 3H), 2.03 (m, 1H), 3.68 (m, 2H), 4.04 (m, 2H), 4.54 (m, 2H, 6.51 (bs, 1H), 7.14-7.25 (m, 5H) ppm.

Chiral HPLC method to separate the E/Z-isomers of 13 and the four isomers of 19: Chiralpak column AD-H, 250 x 4.6 mm ID, particle size 5µm, eluent: n-heptane : 2-butanol 90:10 (v/v), flow: 1.0 mL/min, temperature: 30 °C, UV detection at 220 nm, retention time E-13 = 9.8 min; Z-13 = 10.4 min; 19a-ent1 = 12.7 min; 19b-ent1 = 13.6 min; 19b-ent2 = 15.5 min; 19a-ent2 = 16.4 min.

### Selective hydrogenation of E/Z-mixtures of enamide 13:

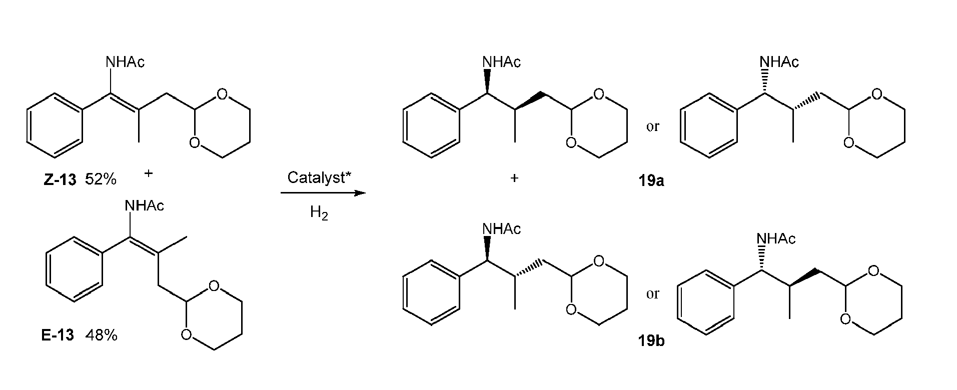

### Selective hydrogenation of Z-13 using phosphoramidites:

Rh(COD)₂BF₄ (4.2 mg, 0.010 mmol, 10 mol%), (S)-monophos (7.2 mg, 0.020 mmol, 20 mol%) and enamide 13 (28.2 mg, 0.102 mmol, Z/E = 52/48) were dissolved in MeOH (5.0 mL) and the resulting solution was stirred under a hydrogen atmosphere (25 bars) at room temperature for 16 hours. The reaction mixture was subsequently filtered over dicalite and concentrated in vacuo to give a yellow oil. ¹H-NMR showed 19% conversion of the Z-isomer and 3% conversion of the E-isomer. The ratio of 19a:19b amounted to 83:17. HPLC (vide supra) showed that 19a had an enantiomeric excess of 19%.

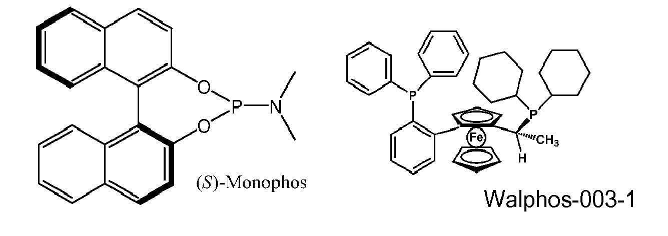

### Selective hydrogenation of E-13 using ferrocene-based ligands:

Rh(COD)₂BF₄ (0.81 mg, 2.0 µmol, 2.0 mol%), (S)-Walphos-003 (1.5 mg, 2.2 µmol, 2.2 mol%) and enamide 13 (27.5 mg, 0.100 mmol, Z/E = 52/48) were dissolved in DCM (5.0 mL) and the resulting solution was stirred under a hydrogen atmosphere (5 bars) at room temperature for 16 hours. The reaction mixture was subsequently filtered over dicalite and concentrated in vacuo to give a yellow oil. ¹H-NMR showed 35% conversion of the Z-isomer and 95% conversion of the E-isomer. The ratio of 19a:19b amounted to 28:72. The enantiomeric excess of 19a was determined to be 71%. The enantiomeric excess of 19b was determined to be 88%.

### Example 13

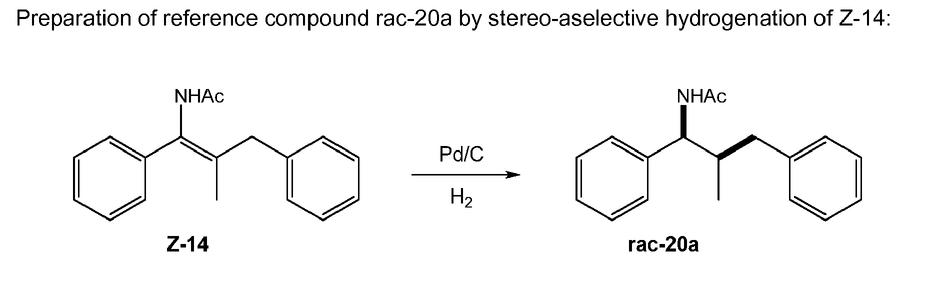

To enamide Z-14 (100 mg, 0.38 mmol) and Pd/C (10 wt%, 35 mg) was added EtOH (2.0 mL) and the resulting mixture was stirred under a hydrogen atmosphere (1 bar) at room temperature for 16 hours. Subsequently, the suspension was filtered over dicalite and concentrated in vacuo to give racemic 20a as an off-white solid (100 mg, 0.38 mmol, quantitative).

¹H-NMR of 20a (CDCl₃, 300 MHz): δ = 0.82 (d, 3H, J = 6.0 Hz), 2.02 (s, 3H), 2.14 (m, 2H), 2.57 (dd, 1H, J = Hz), 4.78 (t, 1H, J = Hz), 6.96-7.24 (m, 10H), 8.14 (bs, 1H) ppm.

Chiral HPLC-method to separate the E/Z-isomers of 14 and the four isomers of 20: column chiralpak OD-H, 250 x 4.0 mm, ID 5µm, eluent: n-heptane : 2-propanol, gradient: from 75:25 (n-heptane : 2-propanol, v/v) to 0:100 over 10 min. (t = 10), maintain 0:100 for 3 min. (t = 13), then to 75:25 in 0.1 min. (t = 13.1), maintain at 75:25 for 6.9 min (t = 20), flow: 1.0 mL/min, temperature: 50 °C, UV-detection at 220 nm, retention time E-14 = 9.38 min.; 20a-ent1 = 9.70 min.; 20a-ent2 = 9.93 min.; 20b-ent1 = 10.30 min.; 20b-ent2 = 10.74 min.; Z-14 = 10.86 min.

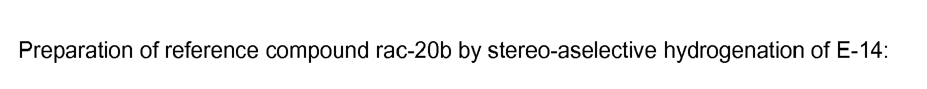

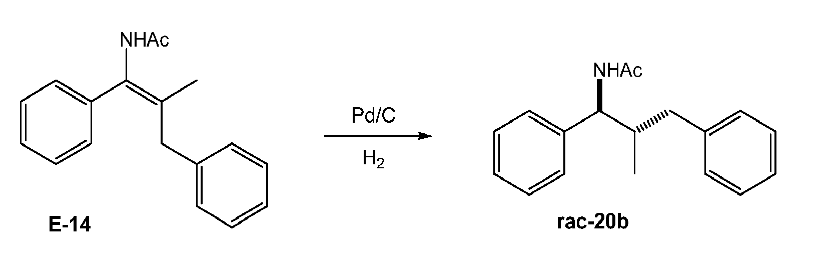

To enamide E-14(100 mg, 0.36 mmol) and Pd/C (10 wt%, 35 mg) was added EtOH (2.0 mL) and the resulting mixture was stirred under a hydrogen atmosphere (1 bar) at room temperature for 16 hours. Subsequently, the suspension was filtered over dicalite and concentrated in vacuo to give racemic 20b as an off-white solid (100 mg, 0.36 mmol, quantitative).

¹H-NMR of 20b (CDCl₃, 300 MHz): δ = 0.63 (d, 3H, J = 6.6 Hz), 1.85 (s, 3H), 2.19 (m, 2H), 2.87 (dd, 1H, J = 3.9, 12.9 Hz), 4.83 (t, 1H, J = 8.7 Hz), 6.35 (bs, 1H), 7.04-7.25 (m, 10H) ppm.

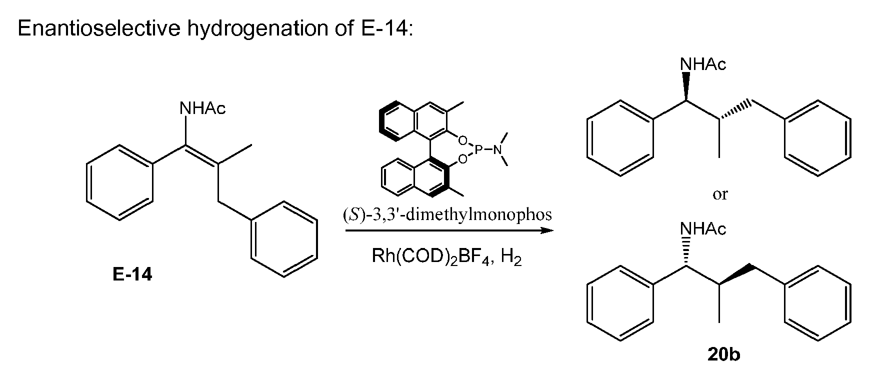

Rh(COD)₂BF₄ (4.1 mg, 10 µmol, 10 mol%), (S)-3,3'-dimethylmonophos (7.7 mg, 20 µmol, 20 mol%) and enamide E-14 (26.4 mg, 99.4 µmol) were dissolved in DCM (5.0 mL) and the resulting solution was stirred under a hydrogen atmosphere (25 bars) at room temperature for 16 hours. The reaction mixture was subsequently filtered over dicalite and concentrated in vacuo to give a yellow oil. ¹H-NMR showed 100% conversion into 20b with a diastereomeric excess of > 99%. Chiral HPLC showed an enantiomeric excess for 20b of 86%.

## Claims

1. A method for the preparation of a compound of formula (I), 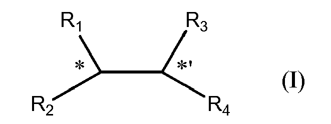 wherein R¹ is different to R²; R³ is different to R⁴; and R¹, R², R³ and R⁴ are each a moiety other than hydrogen, which method comprises,
i) photoirradiating at an intensity above ambient intensity a solution comprising a compound of formula (II), 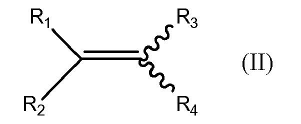 wherein R¹, R², R³ and R⁴ are as defined above, to form a solution of a mixture of compounds of formula (IIa) and (IIb), 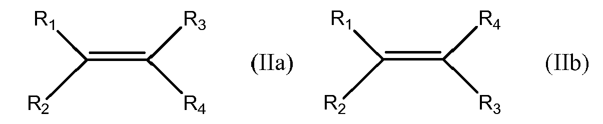 wherein R¹, R², R³ and R⁴ are as defined above; and
ii) contacting the mixture of compounds (IIa) and (IIb) with a hydrogen donating agent in the presence of an asymmetric hydrogenation catalyst, after initiation of i) and before termination of i).

2. A method according to claim 1, which method comprises,
i) photoirradiating continuously at an intensity above ambient intensity a solution comprising a compound of formula (II), 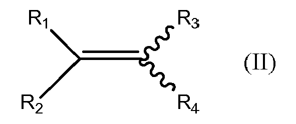 wherein R¹, R², R³ and R⁴ are as defined in claim 1, to form a solution of a mixture of compounds of formula (IIa) and (IIb), 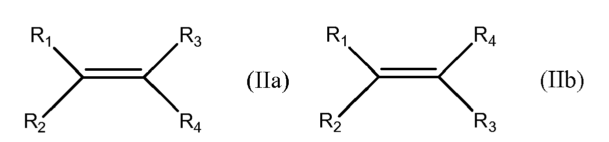 wherein R¹, R², R³ and R⁴ are as defined in claim 1; and simultaneously
ii) contacting the mixture of compounds (IIa) and (IIb) with a hydrogen donating agent in the presence of an asymmetric hydrogenation catalyst.

3. A method according to claim 1, which method comprises, in order,
i) photoirradiating at an intensity above ambient intensity a solution comprising a compound of formula (II), 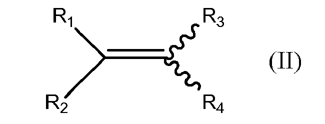 wherein R¹, R², R³ and R⁴ are as defined in claim 1, to form a solution of a mixture of compounds of formula (IIa) and (IIb), 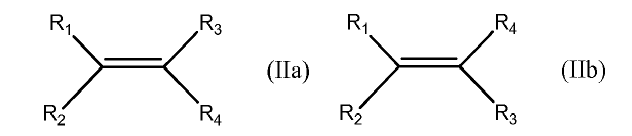 wherein R¹, R², R³ and R⁴ are as defined in claim 1;
ii) contacting the mixture of compounds of formula (IIa) and (IIb) with a hydrogen donating agent in the presence of an asymmetric hydrogenation catalyst; and
iii) repeating at least once i) and ii) in order.

4. A method according to claim 3, wherein i) and ii) are carried out in order three times.

5. A method according to claim 3 or claim 4, wherein i) and ii) are carried out in the same reaction vessel.

6. A method according to any one of claims 1 to 5, wherein a photosensitiser is present in at least i).

7. A method according to any one of claims 1 to 6, wherein the compound of formula (II) is a compound of formula (IIa), 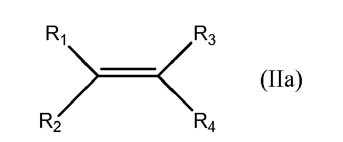 wherein R¹, R², R³ and R⁴ are as defined in claim 1, and the compound of formula (I) is a compound of formula (Ia) or a compound of formula (Ib), 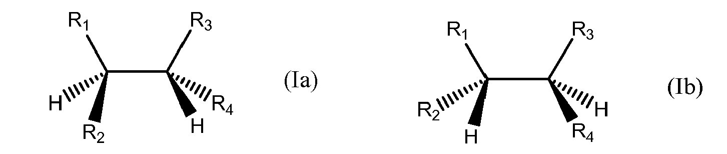 wherein R¹, R², R³and R⁴ are as defined in claim 1.

8. A method according to any one of claims 1 to 7, wherein,
R¹ is -NR⁶C(O)R⁷;
R² is C₁-C₆ alkyl, aryl, heteroaryl, carbocyclyl or heterocyclyl;
R³ is -C(O)R⁵, -S(O)R^{8b}, -SO₂R^{8b}, C₁-C₆ alkyl or aryl;
R⁴ is C₁-C₆ alkyl, aryl, heteroaryl, carbocyclyl or heterocyclyl;
R⁵ is hydroxy, -O⁻, -O⁻Q⁺, C₁-C₆ alkyl, C₁-C₆ alkoxy, C₂-C₆ alkenyl, C₂-C₆ alkynyl, aryl, aryloxy, heteroaryl, carbocyclyl, heterocyclyl or -NR¹⁰₂;
R⁶ is hydrogen, C₁-C₆ alkyl, aryl, heteroaryl, carbocyclyl or heterocyclyl;
R⁷ is hydrogen, C₁-C₆ alkyl, C₁-C₆ alkoxy, aryl, aryloxy, heteroaryl, carbocyclyl or heterocyclyl;
R^{8b} is C₁-C₆ alkyl or C₁-C₆ alkoxy;
R¹⁰ is hydrogen, C₁-C₆ alkyl, aryl, heteroaryl, carbocyclyl or heterocyclyl; and
Q⁺ is a sodium, potassium or lithium ion, or a trialkyl ammonium or tetraalkyl ammonium ion.

9. A method according to any one of claims 1 to 8, wherein,
R¹ is -NR⁶C(O)R⁷;
R³ is -C(O)-O-C₁-C₆ alkyl; and
each of R² and R⁴ is independently C₁-C₆ alkyl, aryl, carbocyclyl or heterocyclyl.

10. A method according to any one of claims 1 to 8, wherein the compound of formula (I) is a compound of formula (I'), the compound of formula (II) is a compound of formula (II'), the compound of formula (IIa) is a compound of formula (IIa'), and the compound of formula (IIb) is a compound of formula (IIb'), 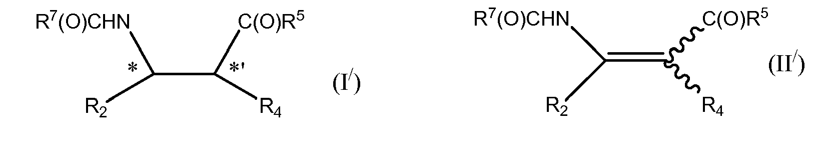 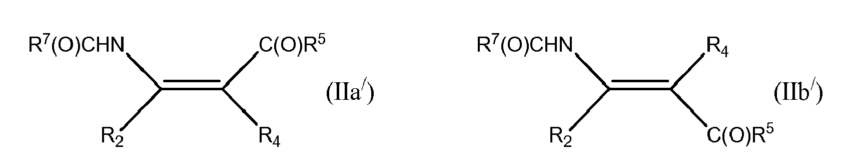 wherein,
R² and R⁴, are each as defined in claim 1; and
R⁵ and R⁷ are each as defined in claim 8; except that,
R² is not NHC(O)R⁷, and R⁴ is not C(O)R⁵.

11. A method according to any one of claims 1 to 8, wherein
R¹ is -NCH(O)R⁷; and
R², R³ and R⁴ are each independently C₁-C₆ alkyl or aryl.

12. A method according to any one of claims 1 to 3, wherein the compound of formula (I) is a compound of formula (I"), the compound of formula (II) is a compound of formula (II"), the compound of formula (IIa) is a compound of formula (IIa"), and the compound of formula (IIb) is a compound of formula (IIb"), 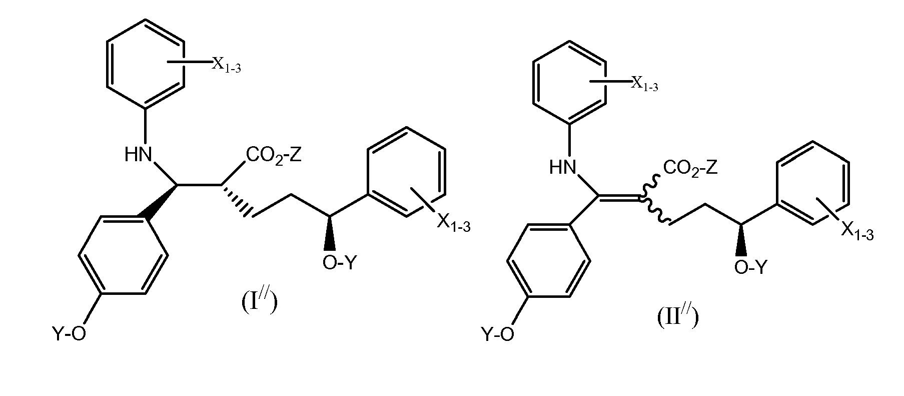 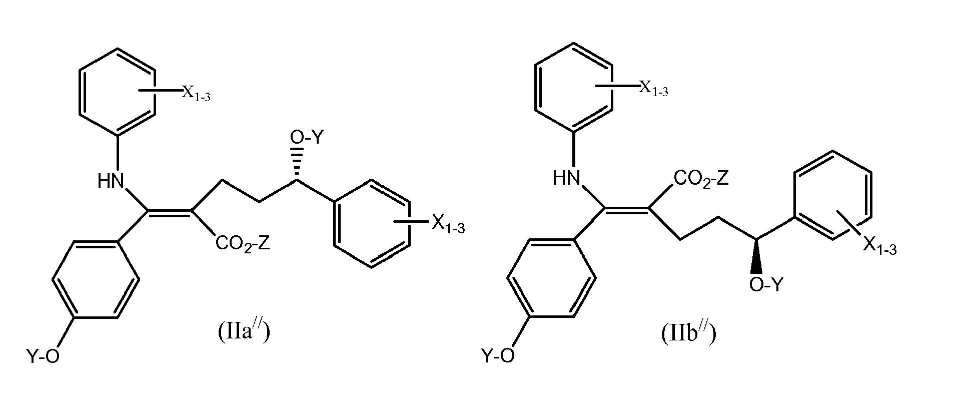 wherein X is a halogen, and each of Y and Z is a protecting group.

13. A method according to claim 12, further comprising cyclising the compound of formula (I^{//}) to form a compound of formula (III), 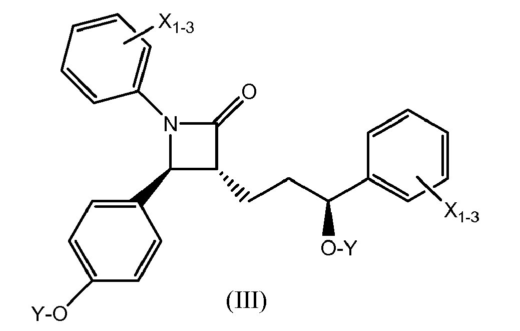 and optionally removing the protecting groups from the compound of formula (III) to form a compound of formula (IV), 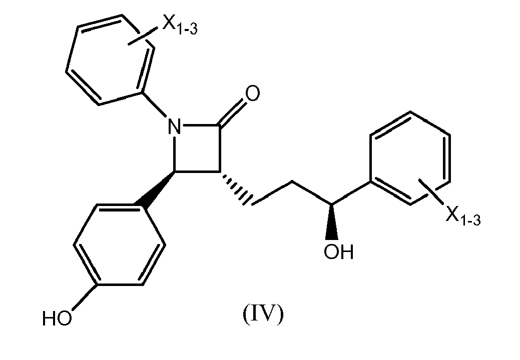 wherein X is as defined in claim 12.

14. A method according to claim 13 wherein the compound of formula (IV) is produced and said compound of formula (IV) is (3R,4S)-1-(4-fluorophenyl)-3-[(3S)-3-(4-fluorophenyl)-3-hydroxypropyl]-4-(4-hydroxyphenyl)azetidin-2-one.
